# EUROPEAN PATENT APPLICATION

(11) **EP 4 600 266 A1**
(43) Date of publication of application: **13.08.2025**
(21) Application number: 23875185.3
(22) Date of filing: 04.10.2023
(51) Int. Cl.: C07K 14/725, C07K 14/54, C07K 14/705, C07K 16/28, C12N 15/62, C12N 5/0783, A61K 35/17, A61K 39/00, A61P 35/02

(54) **CHIMERIC ANTIGEN RECEPTOR TARGETING CD5 AND IMMUNE CELLS EXPRESSING SAME**

(30) Priority: 05.10.2022 KR 20220126846
(71) Applicant: GC Cell Corporation, Yongin-si, Gyeonggi-do 16924 (KR)
(72) Inventor: KIM, Seung min, Yongin-si Gyeonggi-do 16924 (KR); LEE, Eunsol, Yongin-si Gyeonggi-do 16924 (KR); SUN, Hyun seung, Yongin-si Gyeonggi-do 16924 (KR); KIM, Hansol, Yongin-si Gyeonggi-do 16924 (KR); CHO, Sunglim, Yongin-si Gyeonggi-do 16924 (KR); JUNG, Miyoung, Yongin-si Gyeonggi-do 16924 (KR); MIN, Bokyung, Yongin-si Gyeonggi-do 16924 (KR)
(74) Representative: Fish & Richardson P.C.
(86) International application number: PCT/KR2023/015186
(87) International publication number: WO 2024/076121

(57) **Abstract**

The present invention relates to immune cells co-expressing a chimeric antigen receptor comprising an OX40 ligand as an intracellular signaling domain and IL-15, and a composition for preventing or treating cancer comprising the same as an active ingredient. The immune cells of the present invention not only exhibit synergistic tumor cell-killing activity by co-expression of the chimeric antigen receptor and IL-15, but also have significantly improved viability and *in vitro* proliferation rate, and thus they may be used as an efficient anticancer cell therapy. In particular, the immune cells of the present invention, when expressing a chimeric antigen receptor targeting CD5, may be applied as an effective therapeutic composition for various CD5-positive tumors, including lymphocytic leukemia.

## Description

### Technical Field

The present invention relates to immune cells expressing a chimeric antigen receptor which specifically recognizes CD5, and IL-15, and a method of treating a CD5-positive tumor using the same.

### Background Art

Natural killer (NK) cells are lymphoid cells that make up about 10% of blood cells and play an crucial role in immune responses. As a key player in the innate immune defense, NK cell is suitable for adoptive cellular immunotherapy, which kills cancer cells or cells infected with exogenous pathogens. In the field of cell therapy using T cells, another type of immune cells, there have been active studies on technology for introducing a chimeric antigen receptors (CAR) into T cells to increase the specific killing effect against cancer cells expressing specific tumor antigens. CAR, an artificial receptor designed to convey antigen specificity to immune cells, is composed of an extracellular antigen binding domain, a transmembrane domain, and an intracellular signaling domain, which are selected to provide specific immunity by activating immune cells such as T cells. The extracellular antigen binding domain may comprise a single-chain variable fragment (scFv) targeting an identified tumor antigen, thereby triggering the activation of immune cells specific for cancer cells expressing the tumor antigen.

Meanwhile, OX40 ligand (CD252) which belongs to the TNFR superfamily is expressed on antigen-presenting cells (APCs), some natural killer cells, and B cells, within hours to days after activation of these cells. OX40 (CD134), a receptor for OX40 ligand, is expressed on T cells and is expressed on T cells activated by CD28. Expression of OX40 further enhances T cell response induced by CD28 activation, thereby increasing T cell proliferation, cytokine secretion and survival.

CD5, a type I glycoprotein, is a member of the scavenger-receptor family. CD5 is expressed by thymocytes, mature T cells and mature B cells, and is involved in the regulation of activation and differentiation processes of lymphocyte. CD5 mitigate activating signals from the BCR so that B-1 cells can only be activated by very strong stimuli (such as bacterial proteins), not by normal tissue proteins. CD5 is highly expressed in various hematological cancers including acute T lymphocytic leukemia and peripheral T-cell lymphoma, and solid cancers such as breast cancer and thymic cancer, and expression thereof is suppressed in normal cells. Thus, CD5 has been actively studied as a therapeutic target against these tumors.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop efficient immune cell therapy products against various CD5-positive tumors including lymphocytic leukemia. As results, the present inventors have discovered that, when a fusion protein in which a chimeric antigen receptor comprising OX40L as an intracellular signaling domain is linked to IL-15 is expressed in immune cells, the viability, *in vitro* expansion and antitumor activity of the immune cells may all be significantly enhanced, thereby a therapeutically effective amount of the cells may be easily obtained and the cells may be used as an efficient cell therapy product exhibiting an excellent anticancer effect against CD5-positive tumors even at a low dose.

Accordingly, an object of the present invention is to provide a fusion protein comprising: a chimeric antigen receptor comprising an intracellular signaling domain which comprises an OX40 ligand, and an extracellular antigen binding domain that binds to CD5; and IL-15.

Another object of the present invention is to provide immune cells expressing the fusion protein and a composition for preventing or treating CD5-positive tumors comprising the same as an active ingredient.

Other objects and advantages of the present invention will become more apparent from the following detailed description together with the appended claims and drawings.

### Technical Solution

In one aspect of this invention, there is provided a fusion protein comprising:
(a) a chimeric antigen receptor (CAR) comprising an intracellular signaling domain which comprises an OX40 ligand (OX40L), and an extracellular antigen binding domain that binds to CD5; and
(b) interleukin (IL)-15.

The present inventors have made intensive studies to develop efficient immune cell therapy products against various CD5-positive tumors including lymphocytic leukemia. As results, the present inventors have discovered that, when a fusion protein in which a chimeric antigen receptor comprising OX40L as an intracellular signaling domain is linked to IL-15 is expressed in immune cells, the viability, *in vitro* expansion and antitumor activity of the immune cells may all be significantly enhanced, thereby a therapeutically effective amount of the cells may be easily obtained and the cells may be used as an efficient cell therapy product exhibiting an excellent anticancer effect against CD5-positive tumors even at a low dose.

The term "fusion protein" as used herein refers to a recombinant protein molecule in which an amino acid sequence derived from a specific protein or domain is fused to another amino acid sequence derived from a different protein or domain. The amino acid sequences of different origins in the fusion protein may be linked to each other directly, or may be linked to each other via a linker sequence, a tag sequence, a self-cleaving sequence, or a combination thereof. In the fusion protein of the present invention, the amino acid sequence of the chimeric antigen receptor (CAR) and the amino acid sequence of the IL-15 protein may be linked to each other directly or may be linked to each other indirectly via a linker, a tag, self-cleaving sequence, or a combination thereof. The fusion protein may be produced by recombinant technology known in the art or be expressed in target cells.

The term "extracellular antigen binding domain" as used herein refers to a domain that is located in the extracellular portion of the chimeric antigen receptor expressed in target cells (e.g., immune cells), and specifically recognizes a target antigen to activate the apoptotic activity of immune cells in a manner specific to target cells (e.g., cancer cells). For example, the extracellular antigen binding domain may be an antigen-binding fragment of an antibody, such as an Fc receptor or a single-chain variable fragment (ScFv). Accordingly, the term "extracellular antigen binding domain" in the present invention is used in the same sense as the term "extracellular domain", "antigen recognition fragment", or "antigen binding fragment".

According to a specific embodiment of the present invention, the extracellular antigen binding domain is an antigen binding fragment of an anti-CD5 antibody.

The term "antibody" as used herein refers to an antibody against CD5 protein, which specifically recognizes and binds to a specific epitope of the CD5 protein, and is meant to include not only a complete full-length antibody but also an antigen-binding fragment (antibody fragment) of the antibody molecule.

A complete antibody has a structure having two full-length light chains and two full-length heavy chains, wherein each light chain is connected to a heavy chain by a disulfide bond. The heavy chain constant region has gamma (γ), mu (µ), alpha (α), delta (δ) and epsilon (ε) types, and is subclassified into gamma 1 (γ₁), gamma 2 (γ₂), gamma 3 (γ₃), gamma 4 (γ₄), alpha 1 (α₁) and alpha 2 (α₂). The light chain constant region has kappa (κ) and lambda (λ) types.

The term "antigen-binding fragment of an antibody" as used herein refers to a fragment that retains an antigen-antibody binding function in a complete antibody molecule. Specific examples of the antigen-binding fragment include an Fab fragment, an F(ab') fragment, an F(ab')2 fragment, and an Fv fragment.

Among antibody fragments, Fab has a structure having light and heavy chain variable regions, and a constant region of the light chain and the first constant domain (C_{H1}) of the heavy chain, and has one antigen-binding site. Fab' is different from Fab in that it has a hinge region comprising one or more cysteine residues at the C terminus of the C_{H1} domain of the heavy chain. An F(ab')2 antibody is generated by forming a disulfide bond between cysteine residues of the hinge region of Fab'. Fv refers to the smallest antibody fragment only having a heavy chain variable region and a light chain variable region. Two-chain Fv is formed by connecting a heavy chain variable region and a light chain variable region by a non-covalent bond, and single-chain Fv (scFv) may generally have the same structure as a dimer such as two-chain Fv since the heavy chain variable region is connected with the light chain variable region by a covalent bond using a peptide linker, or is directly connected at the C-terminus. Such antibody fragments may be obtained using proteases (e.g., Fab may be obtained by restriction cleavage of a complete antibody with papain, and a F(ab')₂ fragment may be obtained by cleavage with pepsin), or may be produced by gene recombination technology.

More specifically, the antigen-binding fragment of the antibody used in the present invention is an ScFv of the anti-CD5 antibody.

The term "heavy chain" as used herein refers to both a full-length heavy chain which consists of a variable region domain V_{H} and three constant region domains C_{H1}, C_{H2} and C_{H3}, and a fragment thereof, which comprise an amino acid sequence having a sufficient variable region sequence to impart specificity to an antigen. In the present specification, the term "complementarity determining region (CDR)" refers to the amino acid sequence of a hypervariable region of an immunoglobulin heavy or light chain. Three CDRs are included in each heavy chain (HCDR1, HCDR2 and HCDR3) and each light chain (LCDR1, LCDR2 and LCDR3), and these CDRs provide major contact residues for the antibody to bind to an antigen or epitope.

The scope of the antibody or antibody fragment of the present invention includes variants having conservative amino acid substitutions in the CDR regions. In addition, the scope of the antibody or antibody fragment of the present invention may include variants of amino add sequences set forth in the appended Sequence Listing, which are capable of specifically recognizing CD5. For example, the amino acid sequence of the antibody may additionally be modified to further improve the binding affinity and/or other biological properties of the antibody. Such modifications include, for example, deletion, insertion and/or substitution of amino acid residues of the antibody. Such amino acid variations are based on the relative similarity of the amino acid side-chain substituents, for example, their hydrophobicity, hydrophilicity, charge, size, and the like. Analysis of the size, shape and type of the amino acid side-chain substituents reveals that arginine, lysine and histidine are all positively charged residues; alanine, glysine and serine have similar sizes; and phenylalanine, tryptophan and tyrosin have similar shapes. Accordingly, based on these considerations, arginine, lysine and histidine; alanine, glysine and serine; and phenylalanine, tryptophane and tyrosin may be defined as biologically functional equivalents.

Amino acid substitutions in a protein that do not generally alter the activity of the molecule are well known in the art (H. Neurath, R.L.Hill, The Proteins, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, and Asp/Gly.

Considering the above-described variations having biologically equivalent activity, it is interpreted that the antigen-recognizing fragments of the antibody of the present invention and nucleic acid molecules encoding the same also include a sequence showing substantial identity with the sequence set forth in the Sequence Listing. As used herein, the term "substantial identity" means a sequence showing at least 80% homology, at least 85 homology in a specific example, at least 90% homology in another specific example, at least 95% homology in still another example, and at least 99% homology in yet another example, as determined by aligning the sequence of the present invention with any other sequence to correspond to each other as much as possible and analyzing the aligned sequence using an algorithm commonly used in the art. Alignment methods for sequence comparison are known in the art (Smith and Waterman, Adv. Appl. Math. (1981) 2:482; Huang et al. Comp. Appl. BioSci. (1992) 8:155-65 and Pearson et al. Meth. Mol. Biol. (1994) 24:307-31).

According to a specific embodiment of the present invention, the extracellular antigen binding domain comprises a heavy chain variable region comprising HCDR1 having the amino acid sequence of SEQ ID NO: 26, HCDR2 having the amino acid sequence of SEQ ID NO: 28, and HCDR3 having the amino acid sequence of SEQ ID NO: 30.

More specifically, the heavy chain variable region comprises the amino acid sequence of SEQ ID NO: 32.

According to a specific embodiment of the present invention, the extracellular antigen binding domain further comprises a light chain variable region comprising LCDR1 having the amino acid sequence of SEQ ID NO: 34, LCDR2 having the amino acid sequence of SEQ ID NO: 36, and LCDR3 having the amino acid sequence of SEQ ID NO: 38.

More specifically, the light chain variable region includes the amino acid sequence of SEQ ID NO: 40.

Most specifically, the extracellular antigen binding domain that is used in the present invention comprises the amino acid sequence of SEQ ID NO: 7.

According to a specific embodiment of the present invention, the intracellular signaling domain further comprises at least one domain selected from the group consisting of CD28, CD3-zeta, OX40, and 4-1BB. More specifically, the intracellular signaling domain further comprises CD28 and CD3-zeta.

Most specifically, the intracellular signaling domain comprises CD28, OX40L, and CD3-zeta, which are arranged in order from a cell membrane toward the inside of the cell.

According to a specific embodiment of the present invention, the fusion protein further comprises a self-cleaving peptide located between the chimeric antigen receptor and the IL-15. More specifically, the self-cleaving peptide comprises the amino acid sequence of SEQ ID NO: 21.

According to the present invention, the amino acid sequence of SEQ ID NO: 21 is the amino acid sequence of a T2A self-cleaving peptide. The T2A self-cleaving peptide is a type of 2A-self-cleaving peptide that cleaves the full-length protein into short peptide fragments by inducing ribosomal skipping during the intracellular protein translation process. It comprises the 18 amino acid residues of SEQ ID NO: 21 (EGRGSLLTCGDVEENPGP). According to one embodiment of the present invention, a GSG (Gly-Ser-Gly) sequence may be added to the N-terminus to enhance the self-cleavage activity of the amino acid sequence of SEQ ID NO: 21. By the T2A self-cleaving peptide, the chimeric antigen receptor and IL-15 of the present fusion protein may be expressed in cleaved form.

In another aspect of this invention, there is provided a nucleic acid molecule encoding the above-described fusion protein of the present invention.

The term "nucleic acid molecule" as used herein is meant to encompass DNA (gDNA and cDNA) and RNA molecules. The nucleotides which are the basic structural units in nucleic acid molecules, include not only natural nucleotides, but also analogues having modified sugar or base moieties (Scheit, Nucleotide Analogs, John Wiley, New York(1980); Uhlman and Peyman, Chemical Reviews, 90:543-584(1990)). The nucleic acid molecule of the present invention may be inserted into a gene delivery system and introduced into target cells, for example, immune cells, in order to express the above-described fusion protein of the present invention in the target cells.

The term "express" as used herein refers to allowing target cells to express an exogenous gene or artificially introducing an endogenous gene using a gene delivery system to increase the natural expression level of the endogenous gene, thereby making the gene replicable as an extrachromosomal factor or by chromosomal integration in the target cells. Accordingly, the term "expression" is synonymous with "transformation", "transfection" or "transduction".

The term "gene delivery system" as used herein refers to any means for delivering a gene into a cell, and the term "gene delivery" has the same meaning as intracellular transduction of the gene. At the cell or tissue level, gene delivery has the same meaning as spread of the gene. Thus, the gene delivery system of the present invention may be referred to as a gene transduction system or a gene spread system.

To construct the gene delivery system of the present invention, the nucleotide sequence of the present invention is preferably operatively linked to a suitable expression regulatory sequence within a suitable expression construct. In the present specification, the terms "operatively linked" refer to a functional linkage between a nucleic acid expression regulatory sequence (e.g., a promoter, a signal sequence, or an array of transcription regulation factor binding sites) and the target nucleic acid sequence. Through the linkage, the regulatory sequence regulates the transcription and/or translation of the target nucleic acid sequence. The promoter linked to the target gene of the present invention is one that can regulate the transcription of the target gene by action specifically in animal cells, more specifically mammalian cells, most specifically immune cells, and includes, for example, promoters derived from mammalian viruses and promoters derived from mammalian cell genomes. Examples of the promoter include, but are not limited to, mammalian cytomegalovirus (CMV) promoter, adenovirus late promoter, vaccinia virus 7.5K promoter, SV40 promoter, HSV *tk* promoter, RSV promoter, EF1 alpha promoter, metallothionein promoter, beta-actin promoter, human IL-2 gene promoter, human IFN gene promoter, human IL-4 gene promoter, human lymphotoxin gene promoter, and human GM-CSF gene promoter.

The nucleotide sequence of the target gene may be applied to any gene delivery system commonly used for gene introduction. For example, the nucleotide sequence of the target gene may be applied to plasmids, adenoviruses (Lockett LJ, et al., Clin. Cancer Res. 3:2075-2080(1997)), adeno-associated viruses (AAV, Lashford LS., et al., Gene Therapy Technologies, Applications and Regulations Ed. A. Meager, 1999), retroviruses (Gunzburg WH, et al., Retroviral vectors. Gene Therapy Technologies, Applications and Regulations Ed. A. Meager, 1999), lentiviruses (Wang G. et al., J. Clin. Invest. 104(11):R55-62(1999)), herpes simplex viruses (Chamber R., et al., Proc. Natl. Act. Sci USA 92:1411-1415(1995)), vaccinia viruses (Puhlmann M. et al., Human Gene Therapy 10:649-657(1999)), liposomes (Metho s in Molecular Biology, 199, S.C. Basu and M. Basu (Eds.), Human Press 2002), or niosomes.

According to a specific embodiment of the present invention, the gene delivery system used in the present invention is a viral vector. More specifically, the virus is selected from the group consisting of lentiviruses, adenoviruses, adeno-associated viruses (AAVs), retroviruses, herpes simplex viruses, and vaccinia viruses. Most specifically, the virus is a lentivirus.

The list of the nucleotide sequences of nucleic acid molecules encoding the full-length fusion protein of the present invention or each domain of the fusion protein is summarized in Table 2 below.

In still another aspect of this invention, there is provided an immune cell expressing the above-described nucleic acid molecule of the present invention.

In the present specification, the term "immune cell" refers to any type of cell involved in the process of initiating or promoting an immune response, and more specifically, refers to an immune effector cell. Immune cells include, but are not limited to, T cells, B cells, natural killer (NK) cells, natural killer T (NKT) cells, and mast cells. More specifically, the immune cells are natural killer cells.

In still another aspect of this invention, there is provided an immune cell expressing:
(a) a chimeric antigen receptor(CAR) comprising an intracellular signaling domain which comprises an OX40 ligand (OX40L), and an extracellular antigen binding domain that binds to CD5; and
(b) interleukin (IL)-15.

Since the immune cells that are used in the present invention have already been described in detail above, the description thereof will be omitted to avoid excessive overlaps.

The immune cell of the present invention may express the chimeric antigen receptor and IL-15 in the form of a single fusion protein in which they are linked to each other as described above with respect to one aspect of the present invention. Alternatively, the immune cell may express the chimeric antigen receptor and IL-15 as individual protein molecules that are not linked together. In case of latter, the chimeric antigen receptor and IL-15 may be co-transformed into an immune cell by inserting the nucleic acid molecules encoding them into individual gene delivery systems, or they may be inserted together into one gene delivery system and transformed into an immune cell.

According to a specific embodiment of the present invention, immune cells that are used in the present invention are natural killer cells. More specifically, the natural killer cells are natural killer cells frozen and thawed after culturing for 23 to 35 days, more specifically 25 to 33 days, most specifically 28 to 30 days.

In the present specification, the term "culturing" refers to inducing cells to grow and proliferate in an *in vitro* environment such as a culture medium while maintaining their biological activity.

The term "culture medium" as used herein refers to mixtures for efficiently inducing and promoting cell growth and proliferation *in vitro,* which contain essential elements for cell growth and proliferation, such as sugars, amino acids, minerals, and other nutrients. Cell culture media may be optimized depending on the type, phenotype and culture characteristics of specific cell, and include, for example, basal culture media prepared to support cell growth, culture media prepared to promote recombinant production of proteins from cells, and concentrated media prepared by concentrating nutrients at high concentration.

The term "freezing" or "cryopreservation" as used herein refers to maintaining cells stable over a short or long period of time at low temperatures, specifically at ultra-low temperatures of -80°C to -200°C. Cells generally undergo mutation at a rate of about 1 cell per 10,000 cells during the culturing process, and if cells undergo passage over a long period of time, they change to a cell population different from the original one, or may lose their inherent biological activity and function. The risk of infection by mycoplasma also increases in proportion to the period of passages. For this reason, a method of freezing cells is widely used to preserve the inherent characteristics of cells. The present inventors have found that, in culturing immune cells, especially natural killer cells where the efficiency of gene introduction using a lentiviral vector is low, pre-freezing process for an appropriate period of time may greatly improve the gene delivery rate and the cell killing activity. Therefore, the freezing step in the present invention may be performed only for a short period of time, unlike cryopreservation which is performed for the purpose of long-term preservation of cells.

Freezing of the cells may be performed by treating the cells with a cryoprotectant that may minimize cell damage caused by the formation of ice crystals and the imbalance of ions and osmotic pressure, which inevitably occur in the freezing and thawing processes. Freezing medium may contain, for example, dextran, albumin and/or DMSO to improve the safety and stability of cells during freezing.

The term "thawing" as used herein refers to the process of increasing the temperature of frozen or cryopreserved cells until the cells become soft living cells in order to resume their life activities. Thawing may usually be done quickly by removing cryogenically frozen cells from a liquid nitrogen tank or the like and placing the cells in a constant temperature water bath at 37°C.

Since the freezing and thawing processes of the present invention are aimed at improving the efficiency of gene introduction into the cells and enhancing the activity of killing pathogenic cells, rather than long-term storage of the cells, the thawing process may be initiated immediately after completion of freezing without a time interval between freezing and thawing, or if necessary, there may be an appropriate time gap between the freezing process and the thawing process.

In still another aspect of this invention, there is provided a composition for preventing or treating CD5-positive tumors, comprising the above-described immune cells of the present invention as an active ingredient.

In still another aspect of this invention, there is provided a method for preventing or treating CD5-positive tumors, comprising administering the above-described immune cells of the present invention to a subject in need thereof.

The term "treatment" as used herein refers to (a) inhibiting the progress of a disorder, disease or symptom; (b) alleviating the disorder, disease or symptom; or (c) eliminating the disorder, disease or symptom. When the immune cells into which the target gene of the present invention (nucleic acid molecule encoding the chimeric antigen receptor that specifically recognizes CD5) has been introduced are administered to a subject, they function to induce the death of CD5-positive cancer cells, thereby inhibiting the progress of symptoms caused by various CD5-positive tumors, including lymphocytic leukemia, or eliminating or alleviating the symptoms. Thus, the composition of the present invention may serve as a cell therapy composition for the disease alone, or may be administered in combination with other pharmacological ingredients and applied as a therapeutic aid for the disease. Accordingly, as herein used, the term "treatment" or "therapeutic agent" encompasses the meaning of "treatment aid" or "therapeutic aid agent".

The term "administration" or "administering" as used herein refers to injecting a therapeutically effective amount of the composition of the present invention directly into a subject so that the same amount is formed in the subject's body.

The term "therapeutically effective amount" as used herein refers to an amount of the composition sufficient to provide a therapeutic or prophylactic effect to a subject to which/whom the composition of the present invention is to be administered. Accordingly, the term "therapeutically effective amount" is meant to encompass a "prophylactically effective amount".

In the present specification, the term "subject" includes, without limitation, humans, mice, rats, guinea pigs, dogs, cats, horses, cows, pigs, monkeys, chimpanzees, baboons or rhesus monkeys. Specifically, the subject of the present invention is a human.

The term "CD5-positive tumor" as used herein refers to a solid or hematological cancer where CD is highly expressed at a measurable level compared to those in normal cells or CD5-negative tumors. CD5 is a transmembrane receptor protein that is expressed in 85% of T-lineage Acute Lymphoblastic Leukemia (T-ALL) and 75% of peripheral T-cell lymphoma, and is highly expressed in mantle cell lymphoma, B-chronic lymphocytic leukemia (CLL) and hairy cell leukemia. Since CD5 expression is suppressed in normal cells, CD5 is used as an effective target for the treatment and diagnosis of tumors.

CD5-positive tumors that may be prevented or treated by the composition of the present invention include hematological cancers such as leukemia, lymphoma and multiple myeloma, and solid cancers such as breast cancer and thymic cancer, but are not limited thereto and include all malignant tumors that express CD5 at a measurable level on the tumor cell surface thus may be specifically recognized by the immune cells of the present invention.

More specifically, the CD5 positive tumor is T lymphocytic leukemia, and even more specifically, acute T lymphocytic leukemia.

Immune cells of the present invention, specifically natural killer cells, may be comprised in an amount of 10 to 95 wt% based on the total weight of the composition for preventing or treating CD5-positive tumors. In addition, the composition of the present invention may be formulated to further comprise, in addition to natural killer cells, one or more anticancer pharmacological ingredients that exhibit the same or similar functions.

The dose of the composition may be adjusted depending on various factors, including on the type of CD5 positive tumor, the severity of the disease, the types and contents of the active ingredient and other ingredients contained in the composition, the type of formulation, the patient's age, body weight, general health status, gender, diet, the administration time, the route of administration, the secretion rate of the composition, the treatment period, and concurrently used drugs. However, for a desirable effect, the dose of the natural killer cells according to the present invention may be, for example, 0.01 x 10⁷ cells/kg to 1.0 x 10⁹ cells/kg, or 0.5 x 10⁷ cells/kg to 1.0 x 10⁸ cells/kg.

In addition, the composition of the present invention may be administered to a subject by various methods for therapeutic cell injection known in the art. The route of administration may be appropriately selected by a person skilled in the art depending on the mode of administration, the volume and viscosity of body fluid, etc. For example, the composition of the present invention may be administered parenterally, specifically intravenously, subcutaneously or intraperitoneally, and most specifically, intravenously.

### Advantageous Effects

The features and advantages of the present invention will be summarized as follows:
(a) The present invention provides immune cells co-expressing a chimeric antigen receptor comprising an OX40 ligand as an intracellular signaling domain and IL-15, and a composition for preventing or treating cancer comprising the same as an active ingredient.
(b) The immune cells of the present invention not only exhibit synergistic tumor cell-killing activity by co-expression of the chimeric antigen receptor and IL-15, but also have significantly improved viability and *in vitro* proliferation rate, thus may be used as an efficient anticancer cell therapy product.
(c) In particular, when expressing a chimeric antigen receptor targeting CD5, the immune cells of the present invention may be used as an effective therapeutic composition for various CD5-positive tumors including lymphocytic leukemia.

### Brief Description of Drawings

FIG. 1 schematically shows chimeric antigen receptor (CAR) constructs used in the present invention, including a control containing only GFP, a third-generation CAR construct containing CD28, OX40L and CD3ζ as signaling domains, a construct lacking the signaling domains and containing only an IL-15 domain, and a fourth-generation CAR construct in which an IL-15 domain is additionally linked to the third-generation CAR construct.
FIG. 2 shows the results of measuring the viability and fold expansion of third-generation CAR-NK cells and fourth-generation CAR-NK cells in the absence of IL-2 cytokine.
FIG. 3 shows the results of measuring the tumor cell killing ability of third-generation CAR-NK cells and fourth-generation CAR-NK cells against the HER2-positive tumor cell lines HCC1954 and SKOV.
FIG. 4 shows the changes in the amount of IFN-γ secretion by third-generation CAR-NK cells and fourth-generation CAR-NK cells that have come into contact with the HER2-positive tumor cell lines HCC1954 and SKOV.

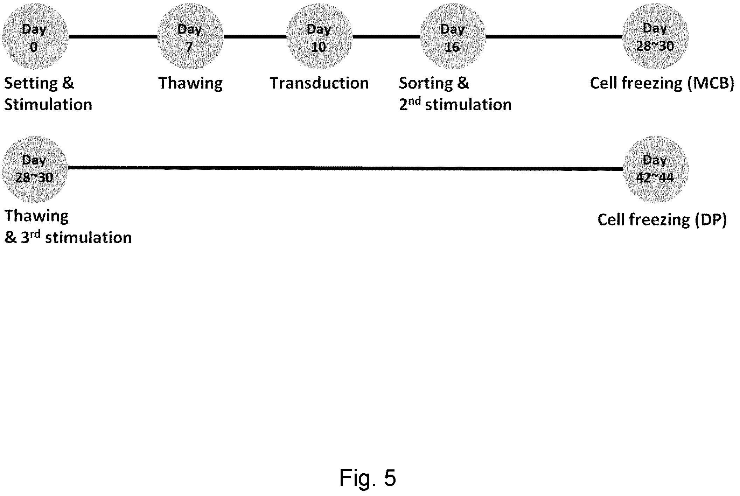

FIG. 5 is a schematic diagram summarizing the NK cell culture process in the present invention.
FIG. 6 shows the results of measuring the expression patterns of CD5 CARs in natural killer cells (MCB) frozen after culturing from day 28 to day 30 of culture through the process shown in the upper portion of FIG. 5, and in natural killer cells (DP) frozen after culturing from day 42 to day 44 of culture through the process shown in the lower portion of FIG. 5.
FIG. 7 shows the results of analyzing the cell phenotypes of MCB and DP based on the expression pattern of each surface marker.
FIG. 8 shows the results of FACS analysis performed to measure CD5 protein expression in each of tumor cell lines of K562, NALM6, CCRF-CEM, RPMI-8402_Luc, NALM6-NucLight, CCRF-CEM-NucLight, and RPMI-8402-NucLight.
FIG. 9 shows the results of measuring the expression levels of CD107a, IFN-γ, and TNFα (n=3) in CBNKs or CD5 CAR-NKs co-cultured with various tumor cell lines.

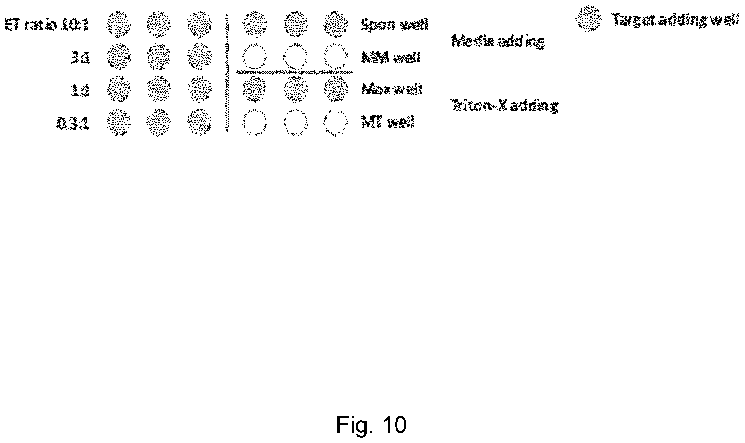

FIG. 10 shows a schematic diagram of culture for evaluating the tumor cell killing ability of CD5 CAR-transduced cells.
FIG. 11 shows the results of evaluating the killing ability of NK cells against CD5-positive and CD-negative tumor cells.
FIG. 12 shows the results of measuring the mean amount of IFN-γ secretion (pg/mL) in three donors.
FIG. 13 shows the results of measuring the mean amount of IL-15 secretion (pg/mL) by NK cells co-cultured with each type of target cells.
FIG. 14 shows the results of evaluating the long-term tumor cell killing ability of CD5 CAR-NK cells after co-culturing with CD5-positive and CD5-negative tumor cells for 96 hours.
FIG. 15 shows the results of measuring the survival rate (%) up to 140 days depending on each administered substance after tumor transplantation in each experimental group.
FIG. 16 shows the bio-imaging up to 112 days following injection of NK cells of the present invention after tumor transplantation.

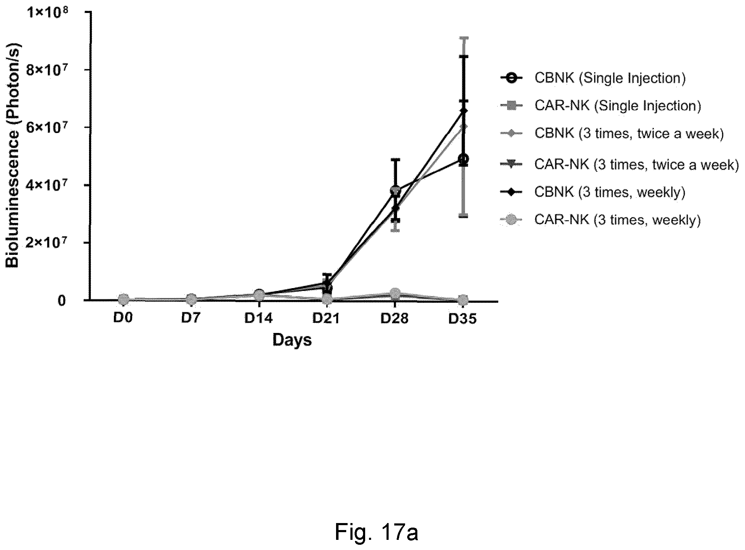

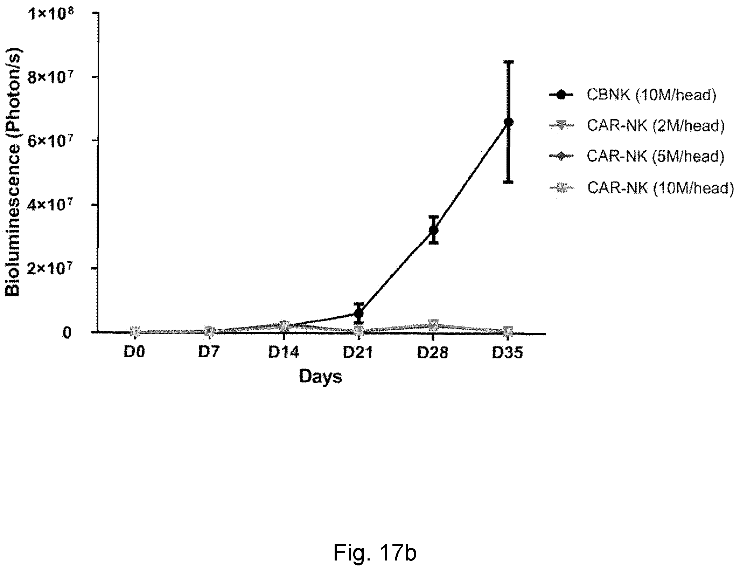

FIG. 17 shows the results of measuring bioluminescence (photons/s) over time until day 35 after tumor transplantation.
FIG. 18 shows the results of measuring the survival rate (%) up to day 133 following injection of NK cells of the present invention after tumor implantation into experimental animals.
FIG. 19 shows the results of bio-imaging up to day 126 following injection of NK cells of the present invention after tumor transplantation into experimental animals.

### Mode for Invention

### Examples

### Example 1: Production of CAR-NK Cells Expressing Third-Generation Chimeric Antigen Receptor (CAR) and Fourth-Generation CAR-NK Cells Co-Expressing Third-Generation Chimeric Antigen Receptor and IL-15 Domain

The structures of chimeric antigen receptors (CARs) used in the present invention are summarized in Table 1 below.

**[Table 1]**

| CAR type | Abbreviation | Signal sequence | ECD | Hinge | TM | Signal-1 | Singal-2 | Signal-3 | 2A peptide | |
|---|---|---|---|---|---|---|---|---|---|---|
| Third generation | HER2 CAR | CD8α | Anti-HER2 scFv | CD8α | CD28 | CD28 | OX40L | CD3z | - | - |
| Truncated | HER2 CAR(t)-IL-15 | CD8α | Anti-HER2 scFv | CD8α | CD28 | - | - | - | T2A | IL-15 |
| Fourth generation | HER2 CAR-IL-15 | CD8α | Anti-HER2 scFv | CD8α | CD28 | CD28 | OX40L | CD3z | T2A | IL-15 |
| Fourth generation | CD5 CAR | CD8α | Anti-CD5 scFv | CD8α | CD28 | CD28 | OX40L | CD3z | T2A | IL-15 |

HER2 CAR (third generation) constructed to evaluate the synergistic effect of co-expression of OX40L-containing CAR and IL-15 of the present invention has a structure in which the following components are sequentially linked together: the signal sequence domain of CD8a (nucleotides 890-952, GenBank NM 001768.6); an anti-HER2 scFv sequence (VH-(GGGGS)3-VL domain); a human CD8α-derived hinge domain (nucleotides 1292-1435, GenBank NM 001768.6); a CD28-derived transmembrane and intracellular signaling domain (nucleotides 679-882, GenBank NM 006139.3); a CD252-derived intracellular signaling domain (nucleotides 141-206, GenBank NM 003326.4); a CD3z-derived intracellular signaling domain (nucleotides 299-634, GenBank NM000734.3); and the stop codon TGA. HER2 CAR(t)-IL-15 has a structure in which the following components are sequentially linked together: the signal sequence domain of CD8α (nucleotides 890-952, GenBank NM 001768.6); an anti-HER2 scFv sequence (VH-(GGGGS)3-VL domain); a human CD8α-derived hinge domain (nucleotides 1292-1435, GenBank NM 001768.6); a CD28-derived transmembrane domain (nucleotides 679-759, GenBank NM 006139.3); a T2A self-cleaving peptide sequence linked to glycine (G)-serine (S)-glycine (G); a human IL-15 sequence (nucleotides 375-860, GenBank NM000585.4); and the TGA stop codon.

HER2 CAR-IL-15 (fourth generation) has a structure in which the following components are sequentially linked together: the signal sequence domain of CD8α (nucleotides 890-952, GenBank NM 001768.6); an anti-HER2 scFv sequence (VH-(GGGGS)3-VL domain); a human CD8α-derived hinge domain (nucleotides 1292-1435, GenBank NM 001768.6); a CD28-derived transmembrane and intracellular signaling domain (nucleotides 679-882, GenBank NM 006139.3); a CD252-derived intracellular signaling domain (nucleotides 141-206, GenBank NM 003326.4); a CD3z-derivied intracellular signaling domain (nucleotides 299-634, GenBank NM000734.3); a T2A self-cleaving peptide sequence linked to G-S-G; a human IL-15 sequence (nucleotides 375-860, GenBank NM000585.4); and the TGA stop codon.

CD5 CAR (fourth generation) has a structure in which the following components are sequentially linked together: the signal sequence of CD8α (nucleotides 890-952, GenBank NM 001768.6); an anti-CD5 scFv domain (VH-(GGGGS)3 linker-VL); a CD8α-derived hinge domain (nucleotides 1292-1435, GenBank NM 001768.6); a CD28-derived transmembrane and intracellular signaling domain (nucleotides 679-882, GenBank NM 006139.3); a CD252-derived intracellular signaling domain (nucleotides 141-206, GenBank NM 003326.4); a CD3z-derived intracellular signaling domain (nucleotides 299-634, GenBank NM000734.3); a T2A self-cleaving peptide sequence linked to G-S-G; a human IL-15 sequence (nucleotides 375-860, GenBank NM000585.4); and the TGA stop codon.

The nucleotide sequence and amino acid sequence of each domain included in the chimeric antigen receptors of the present invention are summarized in Table 2 below.

**[Table 2]**

| SEQ ID NO. | Sequence name | Description of sequence |
|---|---|---|
| 1 | Nucleotide sequence of CD8a | Signal sequence of CD8a (nucleotides 890-952, GenBank NM 001768.6) |
| 2 | Codon-optimized nucleotide sequence of CD8a | Codon-optimized sequence of SEQ ID NO: 1 |
| 3 | Amino acid sequence of CD8a | Amino acid sequence corresponding to SEQ ID NOs: 1 and 2 |
| 4 | Nucleotide sequence of anti-HER2 scFv | Anti- HER2 scFv sequence VH-(GGGGS)3 linker-VL domain |
| 5 | Amino acid sequence of anti-HER2 scFv | Amino acid sequence corresponding to SEQ ID NO: 4 |
| 6 | Nucleotide sequence of anti-CD5 scFv (VH-GS3-VL) | Anti-CDS scFv sequence VH-(GGGGS)3 linker-VL domain |
| 7 | Amino acid sequence of anti-CD5 scFv (VH-GS3-VL) | Amino acid sequence corresponding to SEQ ID NO: 6 |
| 8 | Nucleotide sequence of CD8a | Human CD8a-derived hinge domain (nucleotides 1292-1435, GenBank NM 001768.6) |
| 9 | Codon-optimized nucleotide sequence of CD8a | Codon-optimized sequence of SEQ ID NO: 8 |
| 10 | Amino acid sequence of CD8a | Amino acid sequences of SEQ ID NOs: 8 and 9 |
| 11 | Nucleotide sequence of CD28 | CD28-derived transmembrane and intracellular signaling domain (nucleotides 679-882, GenBank NM 006139.3) |
| 12 | Codon-optimized nucleotide sequence of CD28 | Codon-optimized sequence of SEQ ID NO: 11 |
| 13 | Amino acid sequence of CD28 | Amino acid sequences of SEQ ID NOs: 11 and 12 |
| 14 | Nucleotide sequence of CD252 | CD252-derived intracellular signaling domain (nucleotides 141-206, GenBank NM 003326.4) |
| 15 | Codon-optimized nucleotide sequence of CD252 | Codon-optimized sequence of SEQ ID NO: 14 |
| 16 | Amino acid sequence of CD252 | Amino acid sequences of SEQ ID NOs: 14 and 15 |
| 17 | Nucleotide sequence of CD3z | CD3z-derived intracellular signaling domain (nucleotides 299-634, GenBank NM000734.3) |
| 18 | Codon-optimized nucleotide sequence of CD3z | Codon-optimized sequence of SEQ ID NO: 17 |
| 19 | Amino acid sequence of CD3z | Amino acid sequences of SEQ ID NOs: 17 and 18 |
| 20 | Nucleotide sequence of T2A | T2A self-cleaving peptide sequence |
| 21 | Amino acid sequence of T2A | Amino acid sequence of SEQ ID NO: 20 |
| 22 | Nucleotide sequence of IL-15 | Human IL-15 sequence (nucleotides 375-860, GenBank NM000585.4) |
| 23 | Codon-optimized nucleotide sequence of IL-15 | Codon-optimized sequence of SEQ ID NO: 22 |
| 24 | Amino acid sequence of IL-15 | Amino acid sequences of SEQ ID NOs: 22 and 23 |
| 25 | Nucleotide sequence of anti-CD5 scFv HCDR1 | Nucleotide sequence encoding HCDR1 of anti-CD5 scFv |
| 26 | Amino acid sequence of anti-CD5 scFv HCDR1 | Amino acid sequence of SEQ ID NO: 25 |
| 27 | Nucleotide sequence of anti-CD5 scFv HCDR2 | Nucleotide sequence encoding HCDR2 of anti-CD5 scFv |
| 28 | Amino acid sequence of anti-CD5 scFv HCDR2 | Amino acid sequence of SEQ ID NO: 27 |
| 29 | Nucleotide sequence of anti-CD5 scFv HCDR3 | Nucleotide sequence encoding HCDR3 of anti-CD5 scFv |
| 30 | Amino acid sequence of anti-CD5 scFv HCDR3 | Amino acid sequence of SEQ ID NO: 29 |
| 31 | Nucleotide sequence of anti-CD5 scFv heavy-chain variable region | Nucleotide sequence encoding whole heavy-chain variable region of anti-CD5 scFv |
| 32 | Amino acid sequence of anti-CD5 scFv heavy-chain variable region | Amino acid sequence of SEQ ID NO: 31 |
| 33 | Nucleotide sequence of anti-CD5 scFv LCDR1 | Nucleotide sequence encoding LCDR1 of anti-CD5 scFv |
| 34 | Amino acid sequence of anti-CD5 scFv LCDR1 | Amino acid sequence of SEQ ID NO: 33 |
| 35 | Nucleotide sequence of anti-CD5 scFv LCDR2 | Nucleotide sequence encoding LCDR2 of anti-CD5 scFv |
| 36 | Amino acid sequence of anti-CD5 scFv LCDR2 | Amino acid sequence of SEQ ID NO: 35 |
| 37 | Nucleotide sequence of anti-CD5 scFv LCDR3 | Nucleotide sequence encoding LCDR3 of anti-CD5 scFv |
| 38 | Amino acid sequence of anti-CD5 scFv LCDR3 | Amino acid sequence of SEQ ID NO: 37 |
| 39 | Nucleotide sequence of anti-CD5 scFv light-chain variable region | Nucleotide sequence encoding whole light-chain variable region of anti-CD5 scFv |
| 40 | Amino acid sequence of anti-CD5 scFv light-chain variable region | Amino acid sequence of SEQ ID NO: 39 |

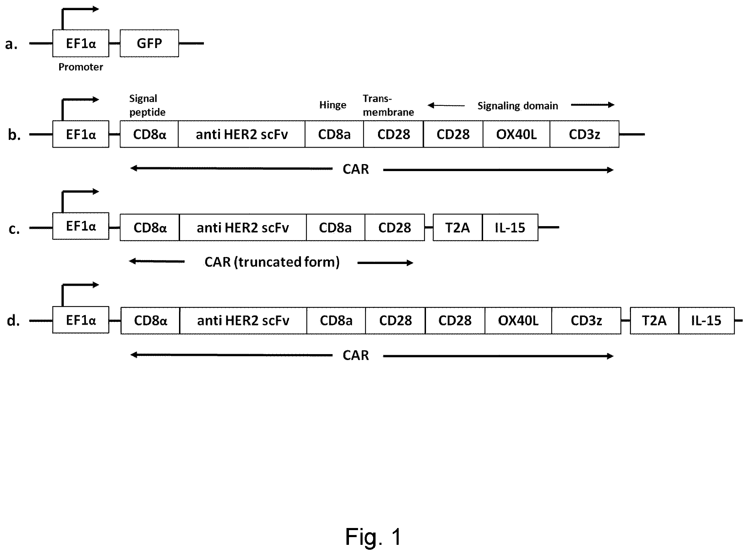

IL-15 is a cytokine that activates NK cells and contributes to the viability and proliferation thereof. Accordingly, to examine whether co-expression of IL-15 in NK cells expressing the chimeric antigen receptor improves the viability and *in vivo* persistence of the NK cells, the present inventors introduced a soluble form of IL-15 protein into a chimeric antigen receptor comprising an scFv fragment, which specifically recognizes HER2, and a signaling domain, and evaluated the efficacy thereof (FIG. 1). NK cells expressing a chimeric antigen receptor in a bicistronic form were produced by linking an anti-HER2 scFv fragment and a signaling domain to a soluble IL-15 domain by a T2A system, and named "fourth-generation CAR-NK cells" ("d" in FIG. 1). As controls, third-generation CAR-NK cells that express a chimeric antigen receptor comprising the anti-HER2 scFv fragment and the signaling domain but does not express the IL-15 domain ("b" in FIG. 1), and NK cells that express the anti-HER2 scFv fragment but lack the intracellular signaling domain and express the IL-15 domain ("c" in FIG. 1; named CAR(t)-IL-15-NK cells) were also produced. The gene expressing GFP was used as a control ("a" in FIG. 1).

### Example 2: Evaluation of Viability and Growth of Third- and Fourth-Generation CAN-NK Cells

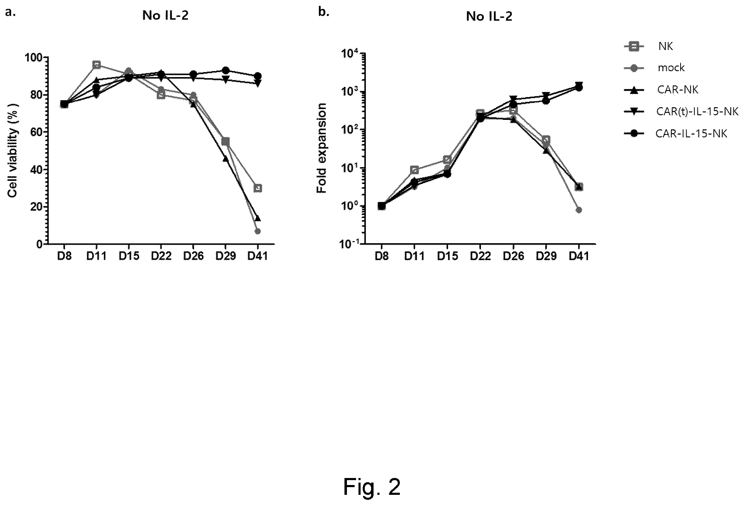

Umbilical cord blood- derived NK cells were cultured and each gene was introduced into the cells on day 7. From day 22 of culture, the viability and growth of the cells were observed while the cells were cultured without adding IL-2 to the culture medium. Cell groups except for the fourth-generation CAR-NK cells and the CAR(t)-NK cells no longer grew with a significant decrease in the viability thereof starting from the time IL-2 was not added, and most of the cells died on day 41 of culture (FIG. 2). It could be confirmed that only CAR-NK cells with the structure comprising the IL-15 domain maintained their viability, implying that and the secreted IL-15 contributed to the survival and proliferation of the NK cells.

### Example 3: Comparison for Efficacy of Third-Generation and Fourth-Generation CAR-NK Cells and Synergistic Effect Induced by IL-15 Domain

### 3-1) Evaluation of Tumor Cell Killing Ability

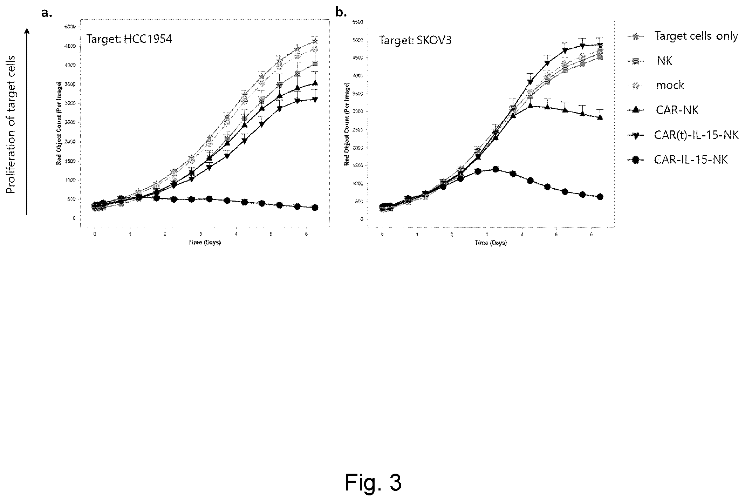

To evaluate the efficacy of the fourth-generation CAR-NK cells, the NK cells were co-cultured with a tumor cell line expressing HER2 to measure the cytotoxicity of the NK cells. Two tumor cell lines (HCC1954 and SKOV3) expressing HER2 were used as targets. RFP was expressed such that the fluorescence signal disappeared when the cells died. From day 22 of culture, various types of NK cell groups and tumor cell lines cultured in the absence of IL-2 were co-cultured for 6 days at a ratio of E: T = 0.3: 1, and then the proliferation of the tumor cells was checked. The fourth-generation HER2 CAR-NK cells secreting IL-15 in the two tumor cell lines (HCC1954 and SKOV3) exhibited excellent cytotoxicity by inhibiting the proliferation of the tumor cell lines, whereas the third-generation HER2 CAR-NK cells and HER2 CAR(t)-NK cells showed relatively low cytotoxicity (FIG. 3). The third-generation HER2 CAR-NK cells appeared to fail to effectively inhibit the formation of tumor cells due to the absence of additional cytokines such as IL-2, and it could be assumed that the HER2 CAR(t)-NK cells exhibited a low cytotoxic effect due to the absence of the signaling domain.

### 3-2) Evaluation of IFN-γ Secretion

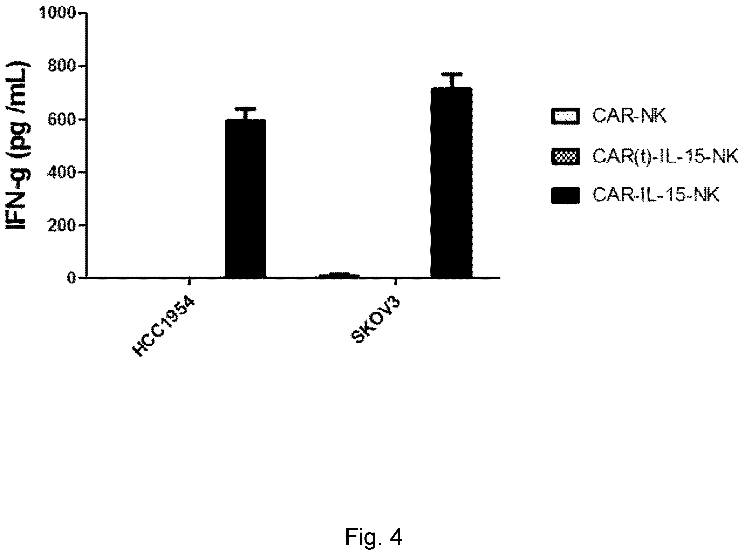

As a result of measuring the secretion of IFN-γ, the functional cytokines secreted by NK cells when the NK cells come into contact with target tumor cells, it was observed that the third-generation HER2 CAR-NK cells and HER2 CAR(t)-NK cells secreted little or no IFN-γ, whereas the fourth-generation CAR-NK cells secreted a very high level of IFN-γ (FIG. 4). Thereby, it could be confirmed that, contrary to the third-generation CAR-NK cells which do not survive and proliferate smoothly, the fourth-generation CAR-NK cells exhibited a synergistic antitumor effect due to co-expression of IL-15 and the chimeric antigen receptor comprising OX40L as a signaling domain.

### Example 4: Culture of NK Cells and Freezing of Cells at Different Time Points of Culture

### 4-1) Cell Thawing

The frozen cells were quickly thawed in a preheated constant temperature water bath at 37°C. When the degree of thawing reached 90% or more, the cells were transferred into a 50-mL tube on a biological safety cabinet, and a 10-fold volume (v/v) of ACD buffer was slowly added thereto. Next, centrifugation was performed at 1,200 rpm and 4°C for 10 minutes, the supernatant was completely removed, and then the cells were suspended in CellGro medium and counted.

### 4-2) Culture of NK Cells and Transduction with Lentiviral Vector

On day 0 of culture, 1.0 x 10⁶ cells/mL of NK cells, 2.5 x 10⁶ cells/mL of feeder cells, and CellGro medium were mixed together at a ratio of 1:1:1, followed by stationary culture in a CO₂ incubator at 37°C. Cellgro medium supplemented with IL-2 (1,000 IU/mL), human plasma (2%), and OKT3 (10 µg/mL) corresponding to the total volume was used. On day 4 of culture, CellGro mixed medium containing IL-2 (1,000 IU/mL) and human plasma (1%) was added in an equal amount to the total volume. On day 7 of culture, all of the cells were recovered and counted without separate dilution, and when the cell count was more than 0.66 x 10⁶ cells/mL, the cells were frozen. On day 7 of culture, thawed raw cells were suspended in CellGro medium and counted, and the cells diluted at a concentration of 1.0 x 10⁶ cells/mL were cultured. On day 10 of culture, to treat 1.0 x 10⁶ cells/mL of cells with 20 MOI of a lentiviral vector, the cells were treated with mixtures including 6.6 x 10⁸ TU/mL of the lentiviral vector (30 µL). The compositions of the mixtures for lentiviral vector transduction are shown in Table 3 below.

**[Table 3] Composition of each solution for lentiviral vector transduction**

| Mixture | Solution | Storage amount | Final concentration |
|---|---|---|---|
| 1 | Lentiviral vector | Not applicable | 20 MOI/1 x 10⁶ cells |
| | Protransduzin RUO | 10 mg/mL in DMSO | 10µg/mL |
| 2 | IL-21 | 100 µg/mL in PBS | 20 ng/mL |
| | Human plasma | Not applicable | 1% |
| | IL-2 | 2,500,000 IU/mL | 1,000 IU/mL |
| | Glutamine | 200 mM | 2% |
| | CellGro medium | Not applicable | Not applicable |

Mixtures 1 and 2 were prepared separately and then slowly added to the culture vessel. A mixed medium obtained by mixing 1 mL of CellGro medium with human plasma (1%) and IL-2 (1,000 IU) (hereinafter referred to as CellGro mixed medium) was added to CBNK that did not undergo lentiviral vector transduction, followed by stationary culture in a CO₂ incubator at 37°C. On day 11 of culture, CellGro mixed medium of same volume as those on day 10 based on 6-well, and IL-21 at a concentration of 20 ng/mL, were added. On day 13 of culture, cells were recovered and adjusted to 1 x 10⁶ cells/mL by adding CellGro mixed medium without further dilution. On day 16 of culture, CD5-positive cells were isolated to obtain NK cells expressing CD5 CAR. To this end, all NK cells were recovered and centrifuged at 1,200 rpm and 4°C for 5 minutes, and the supernatant was removed. Then, the cells were suspended in MACS buffer and then counted. MACS buffer was added to make 1.0 x 10⁷ cells/mL. Next, biotin-tagged recombinant human CD5 protein (His & AVI tag) (250 µg/mL) was added to the cells in an amount of 1 µL/10⁶ cells and mixed well, followed by incubation at 4°C for 30 minutes. A 10-fold volume of MACS buffer was added to the first incubation mixture, followed by centrifugation at 340g and 4°C for 10 minutes, and the supernatant was removed. After the cells were suspended in MACS buffer to 1.0 x 10⁸ cells/mL, anti-biotin beads (Miltenyi Biotec, 130-092-357) were added thereto in an amount of 37.5 µL/40 x 10⁶ cells, followed by incubation at 4°C for 15 minutes. A 10-fold volume of MACS buffer was added thereto, followed by centrifugation at 340g and 4°C for 10 minutes, and then the supernatant was removed. After adding MACS buffer to become 1.0 x 10⁸ cells/500 µL, the cells were well lysed by pipetting several times. An LS column (Miltenyi Biotec) was mounted on a QuadroMACS^{™} separator (Miltenyi Biotec), and 3 mL of MACS buffer was flowed through the column to activate the column. The cells incubated with the beads were well lysed by pipetting, and 500 µL of the cell suspension was loaded onto the column and kept until the cell suspension flowed down. To wash the column, the column was washed a total of three times by flowing 3 mL of MACS buffer therethrough. After separating the column from the QuadroMACS^{™} separator, 5 mL of MACS buffer was added and CD5 CAR-positive cells were recovered by pressing with the plunger. The isolated cells were centrifuged, and then CellGro mixed medium was added thereto to reach a cell concentration of 1 x 10⁶ cells/mL. Then, re-stimulation using feeder cells was performed in the same manner as that on day 0 of culture, followed by stationary culture in a CO₂ incubator at 37°C. On day 19 of culture, the same amount of CellGro mixed medium as the culture volume on day 16 of culture was added. On day 21 of culture, the cells were recovered and counted without separate dilution, and the cells were adjusted to 1 x 10⁶ cells/mL by adding CellGro mixed medium. Next, the cells were cultured while being maintained at a concentration of 1 x 10⁶ cells/mL by adding CellGro mixed medium during day 28 to 30 of culture. All NK cells on days 28 to 30 of culture were recovered and then frozen. The cells frozen after day 28 to 30 of culture are hereinafter referred to as "MCB". After adding CellGro medium to the remaining cells to reach a cell concentration of 1.0 x 10⁶ cells/mL, re-stimulation using feeder cells was performed in the same manner as that on day 16 of culture, followed by stationary culture in a CO₂ incubator at 37°C. Next, the cells were cultured while being maintained at a concentration of 1 x 10⁶ cells/mL by adding CellGro mixed medium for 14 days. All NK cells on days 42 to 44 of culture were recovered and then frozen, and the frozen cells were named "DP".

### 4-3) Cell Freezing

After centrifugation, the cells were suspended in PBS, mixed with a freezing medium mixture at a ratio of 1:1, dispensed in each freezing container at a concentration of 100 x 10⁶ cells/mL, and then frozen using a controlled-rate freezer (CRF).

### Example 5: Characterization of CD5 CAR-Transduced NK Cells

### 5-1) Identification of CD5 CAR Expression

1 to 5 x 10⁵ cells were collected and centrifuged, and the supernatant was removed. Then, the cells were suspended in FACS buffer supplemented with 2 mL of 2% FBS. Next, the cell suspension was centrifuged, and the supernatant was removed. Next, 100 µL of FACS buffer and 1 µL of biotin-tagged recombinant human CD5 protein (His & AVI tag) (250 µg/mL) were added to the cells, followed by incubation under a light-shielded condition at 4°C for 30 minutes. Thereafter, 2 mL of FACS buffer was added to the cells, followed by centrifugation, and then the supernatant was removed. For second staining, 100 µL cell buffer was added to the washed NK cells, and then antibodies to be used for analysis were added thereto, followed by incubation under a light-shielded condition at 4°C for 30 minutes.

**[Table 4] Antibodies for detecting CD5 CAR-NK expression**

| Antibody | Amount |
|---|---|
| Streptavidin-PE | 0.5 µL |
| Anti-human CD56 APC | 5 µL |
| Anti-human CD3-PE-Cy7 | 1 µL |
| 7-AAD | 5 µL |
| FACS buffer | To make 100 µL |
| Total amount | 100 |

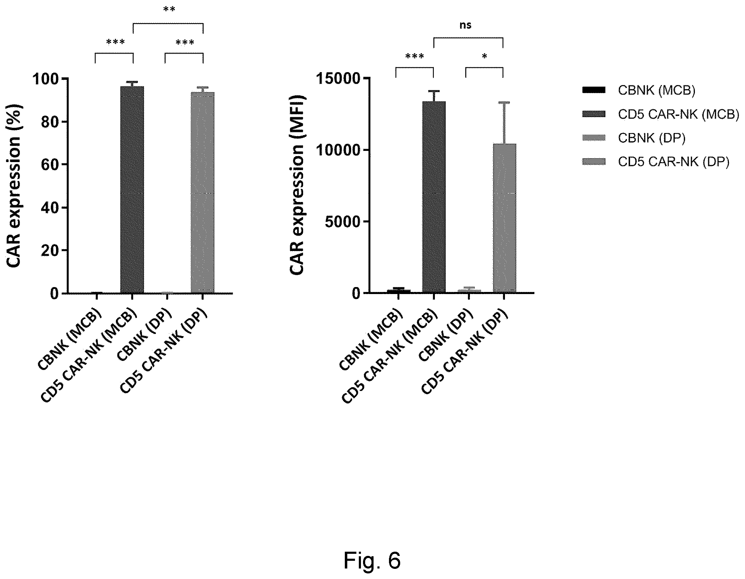

Next, 2 mL of FACS buffer was added to the cells, followed by centrifugation, and the supernatant was removed. Then, 200 µL of BD Cytofix solution was added to the cells and mixed well, and then the stained cells were analyzed using the LSRfortessa system. Raw data were analyzed using the Flowjo program, and gating was performed in the following order: "singlets → lymphocytes → living cells (7-AAD-) → NK cells (CD56+, CD3-) → CD5 CAR expression". After thawing, the CD5 CAR expression rate of MCB was 96.46±2.00 (mean ± standard deviation), and the CD5 CAR expression rate of DP was 93.68±2.23 (mean ± standard deviation), indicating that both MCB and DP cells showed a CD5 CAR expression rate of 90% or more (FIG. 6). The statistical significance of these values was assessed through a two-tailed t-test (Table 5, *p<0.05, **p<0.01, ***<0.001, ns: not significant). From the above results, it was confirmed that the expression of CD5 CAR was well maintained even when the culture period was increased, and that the expression of CD5 CAR did not decrease rapidly after the frozen cells were thawed.

**[Table 5] CAR expression of MCB and DP**

| **CAR expression** | | % | MFI |
|---|---|---|---|
| CBNK (MCB) | Mean | 0.19 | 233.65 |
| | STD | 0.08 | 101.10 |
| CD5 CAR-NK (MCB) | Mean | 96.46 | 13437.68 |
| | STD | 2.00 | 482.37 |
| p-value of CBNK (MCB) vs CDSCAR-NK (MCB) | | 0.0001 | 0.0004 |
| CBNK (DP) | Mean | 0.26 | 245.49 |
| | STD | 0.14 | 136.91 |
| CD5 CAR-NK (DP) | Mean | 93.68 | 10443.52 |
| | STD | 2.23 | 2875.77 |
| p-value of CBNK (DP) vs CDSCAR-NK (DP) | | 0.0002 | 0.0232 |
| p-value of CDSCAR-NK (MCB) vs CDSCAR-NK (DP) | | 0.0093 | ns |

### 5-2) Phenotype of CD5 CAR-Transduced NK Cells

NK cells were suspended in FACS buffer at a concentration of 1.0 to 2.5 10⁶ cells/mL. 100 µL of the cell suspension was dispensed into each well of a plate where an antibody mixture has been dispensed. The cells and antibodies were mixed well by pipetting several times. The composition of the antibody mixture is shown in Table 6 below. PE antibody corresponding to each phenotype was added.

**[Table 6] Composition of antibody mixture for phenotypic staining**

| | **PE_Cy7** | | **PE** | | **APC** | | **PerCP_Cy5.5** | | Total staining volume (µL) |
|---|---|---|---|---|---|---|---|---|---|
| | PE_Cy7 | Volume (µL) | PE | Volume (µL) | APC | volume (µL) | PerCP_C y5.5 | volume (µL) | |
| 1 | CD3 | 1 | CD16 | 5 | CD56 | 5 | 7-AAD | 5 | 16 |
| 2 | | | NKG2A | 1 | | | | | 12 |
| 3 | | | NKG2C | 1 | | | | | 12 |
| 4 | | | NKG2D | 5 | | | | | 16 |
| 5 | | | NKp30 | 1 | | | | | 12 |
| 6 | | | NKp44 | 5 | | | | | 16 |
| 7 | | | NKp46 | 5 | | | | | 16 |
| 8 | | | CXCR3 | 5 | | | | | 16 |
| 9 | | | DNAM-1 | 5 | | | | | 16 |
| 10 | | | CD25 | 5 | | | | | 16 |
| 11 | | | CD57 | 1 | | | | | 12 |
| 12 | | | CD62L | 1 | | | | | 12 |
| 13 | | | CD69 | 5 | | | | | 16 |
| 14 | | | PD-1 | 1 | | | | | 12 |
| 15 | | | TIGIT | 1 | | | | | 12 |
| 16 | | | LAG3 | 1 | | | | | 12 |
| 17 | | | TIM3 | 1 | | | | | 12 |
| 18 | | | KLRG-1 | 1 | | | | | 12 |
| 19 (FMO1) | | | mIgG1 | 5 | | | | | 16 |
| 20 (FMO2) | | | mIgG2a | 5 | | | | | 16 |
| Subtype | mIgG 1 | 1 | mIgG1 | 5 | mIgG1 | 5 | | | 16 |

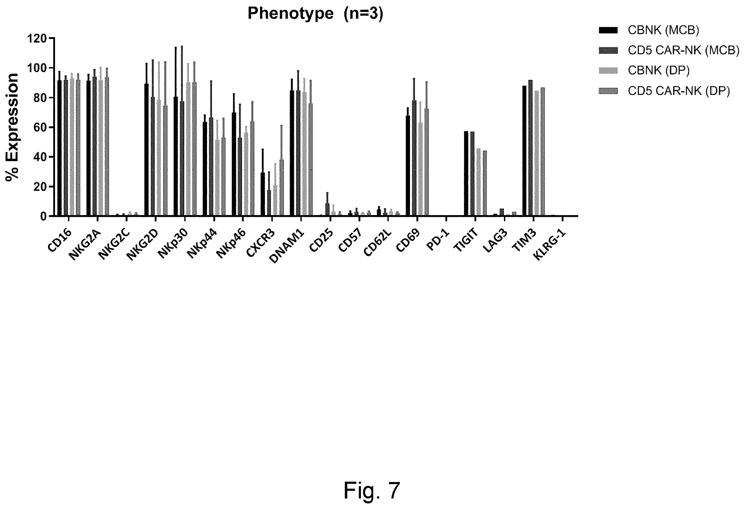

After incubation at 4°C for 30 minutes, 100 µL of FACS buffer was added to the cells and mixed by pipetting, followed by centrifugation (at 2,000 rpm for 3 minutes at 4°C). 200 µL of BD Cytofix solution was dispensed into each well. Flow cytometry was performed using the LSRfortessa system and then the results were analyzed. NK cells were gated in the following order: singlets → lymphocytes → living cells (7-AAD-) → NK cells (CD56+, CD3-). The expression of each marker was calculated based on the subtype antibody. To characterize the NK cells, the expression of 18 surface markers was analyzed by flow cytometry, and the MCB and DP of CD5 CAR-NK were compared with the MCB and DP of CBNK. As a result, the expression of each surface marker in CD5 CAR-NK was not significantly different from that in CBNK and was also not significantly different between MCB and DP (FIG. 7 and Table 7).

**[Table 7] Phenotype values (%) of NK cells from three donors (2024P, 2044P, and 605463p)**

| | 2024P | | | | 2044P | | | | 605463P | | | |
|---|---|---|---|---|---|---|---|---|---|---|---|---|
| | MCB | | DP | | MCB | | DP | | MCB | | DP | |
| | CBNK | CD5 CAR-NK | CBNK | CD5 CAR-NK | CBNK | CD5 CAR-NK | CBNK | CD5 CAR-NK | CBNK | CD5 CAR-NK | CBNK | CD5 CAR-NK |
| CD16 | 89.79 | 91.7 | 92.25 | 89.28 | 87.12 | 89.67 | 89.89 | 90.35 | 97.94 | 94.37 | 96.28 | 96.18 |
| NKG2A | 93.4 | 98.08 | 96.12 | 97.39 | 94.03 | 94.99 | 97.14 | 96.74 | 86.71 | 89.09 | 81.82 | 86.76 |
| NKG2C | 0.96 | 1.48 | 2.73 | 1.88 | 1.01 | 0.53 | 1.89 | 1.79 | 0.73 | 0.79 | 0.73 | 0.58 |
| NKG2D | 98.55 | 98.42 | 97.41 | 96.59 | 95.4 | 90.59 | 88.66 | 85.77 | 73.91 | 52.57 | 49.95 | 41.41 |
| NKp30 | 99.76 | 99.55 | 97.92 | 97.61 | 99.76 | 98.12 | 97.07 | 98.2 | 42.85 | 35 | 75.93 | 74.56 |
| NKp44 | 65.79 | 92.6 | 57.42 | 65.82 | 58.57 | 44.54 | 60.48 | 53 | 66.76 | 62.88 | 36.77 | 40.01 |
| NKp46 | 73.71 | 49.39 | 57.08 | 68.13 | 80.27 | 77.01 | 59.93 | 74.58 | 56.05 | 32.43 | 52.26 | 49.2 |
| CXCR3 | 47.11 | 28.07 | 37.33 | 49.35 | 18.17 | 20.64 | 13.19 | 53.21 | 23.37 | 4.7 | 12.87 | 11.74 |
| DNAM1 | 87.21 | 94.26 | 87.2 | 83.64 | 90.68 | 90.32 | 90.6 | 86.1 | 76.78 | 70.19 | 73.98 | 58.45 |
| CD25 | 0.52 | 13.58 | 0.67 | 0.52 | 0.27 | 0.61 | 0.85 | 0.19 | 0.24 | 12 | 8.14 | 3.15 |
| CD57 | 3.23 | 5.56 | 2.51 | 3.22 | 2.51 | 1.81 | 1.88 | 2.37 | 1.07 | 1.36 | 1.76 | 1.54 |
| CD62L | 6.31 | 0.42 | 2.38 | 1.64 | 4.62 | 5.27 | 2.73 | 1.9 | 1.89 | 1.36 | 4.82 | 2.78 |
| CD69 | 68.98 | 93 | 62.65 | 86.79 | 72.49 | 64.23 | 77.04 | 78.24 | 62.56 | 77.39 | 49.75 | 52.44 |

### Example 6: Evaluation of In Vitro Efficacy of CD5 CAR-Transduced Cells

### 6-1) Generation and Culture of Tumor Cell Lines

Frozen K562, NALM6, CCRF-CEM, RPMI-8402 Luc, NALM6-NucLight, CCRF-CEM-NucLight, or RPMI-8402-NucLight cells were thawed quickly in a constant-temperature water bath at 37°C and then diluted in 10 mL of culture medium. The cells were centrifuged, the supernatant was removed, and then the cells were suspended in 1 mL of medium and counted. 1 to 2 x 10⁶ cells were placed in a T75 flask and cultured in a CO₂ incubator for 2 or 3 days. To generate NucLight-labeled tumor cell lines, cell lines were transduced with Incucyte^{®} NuclightRed lentivirus (Sartorius, Gottingen, Germany) using 8 µg/mL polybrene (Santa Cruz, CA, USA). After for 4 days, puromycin (Gibco, USA) was treated at 0.5, 1, 1.5 and 2 µg/mL. 7 days after puromycin treatment, the lowest concentration that killed 100% of tumor cells was selected, and then red fluorescent protein- expressing cells were selected and cultured.

### 6-2) CD5 Expression in Tumor Cells

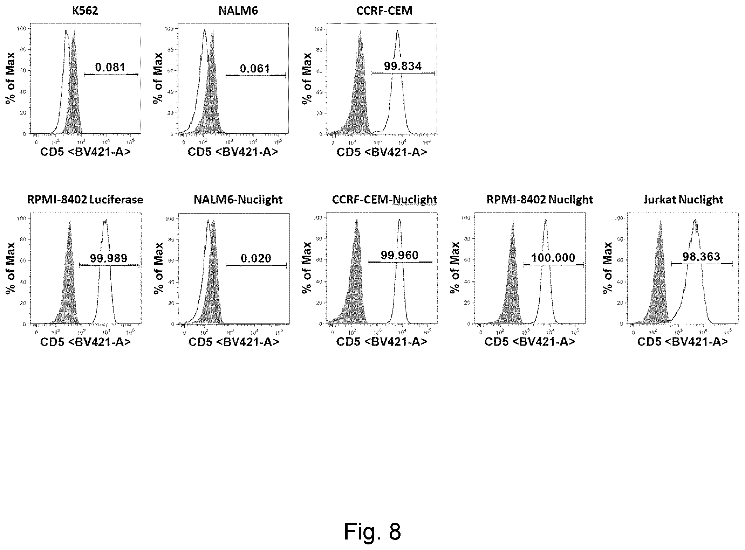

1 to 5 x 10⁵ of recovered tumor cells were transferred into a FACS tube. After centrifugation, the cells were added to 100 µL of FACS buffer mixed with 1 µL of anti-human CD5 PE, and incubated at 4°C under a light-shielded condition for 30 minutes. 1 mL of FACS buffer was added to the cells, followed by centrifugation, and then the supernatant was removed. 200 µL of BD Cytofix solution was added and mixed with the cells, and then FACS analysis was performed. In FIG.8, the gray shade indicates the subtype control, the blue solid line shows the result of anti-human CD5 antibody staining, and the number represents the expression rate (%) compared to the subtype control. It was confirmed that K562, NALM6, and NALM6-Nuclight barely expressed CD5, whereas CCRF-CEM, CCRF-CEM-Nuclight, RPMI-8402_Luc, RPMI-8402-Nuclight, and Jurkat-Nuclight highly expressed CD5 (FIG. 8).

### 6-3) Expression of CD107a and Cytokines on CD5 CAR-Transduced Cells

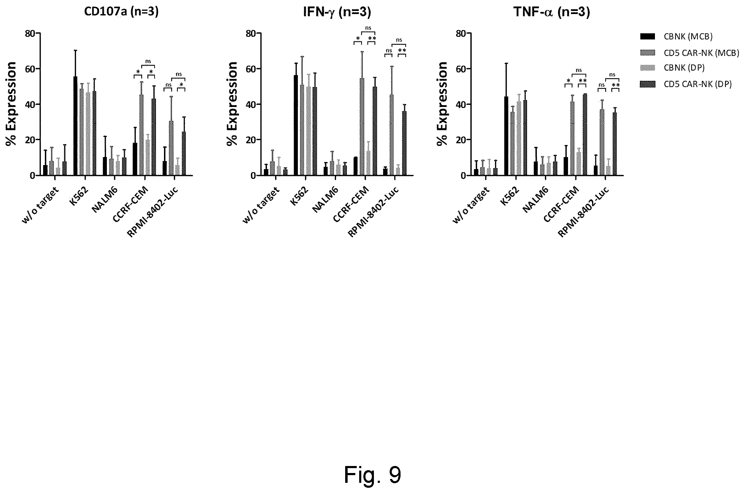

Each of NK cell and target tumor cell lines was suspended in RPMI-1640 + 10% FBS (hereinafter referred to as assay medium) at 2.5 x 10⁶ cells/mL. In order to check whether CD5-specific cell activity would appear, CCRF-CEM and RPMI-8402_Luc were used as CD5-positive tumor cell lines, and NALM-6 was used as a CD5-negative tumor cell line. Golgistop (1.3 µ/mL) and Golgiplug (2 µ/mL) were added to and mixed with cells to prevent the cytokines produced within NK cells from being secreted extracellularly. APC anti-human CD107a antibody (1 µL) was dispensed into the (-) well and target well of a 96-well round bottom plate. To identify the cytokine intrinsically expressed by NK cells, 100 µL of assay medium and 100 µL of NK cells were added to the (-) well of the plate into which the antibody had been dispensed, and 100 µL of NK cells and 100 µL of a target tumor cell line were added to the target well. After the 96-well plate was wrapped in foil to protect it from light, and the cells were co-cultured in a CO₂ incubator at 37°C for 4 hours. After centrifugation (at 2,000 rpm for 3 min at 4°C), the supernatant was removed, and 200 µL of FACS buffer was added and mixed with the cells, followed by centrifugation, and the supernatant was removed. For cell surface staining, anti-human CD3 PerCP-Cy5.5 (1 µL), anti-human CD56-APC-e780 (1 µL), and 7-AAD (4 µL) were added to 100 µL of FACS buffer per well, followed by incubation at 4°C for 30 minutes. After adding 100 µL of FACS buffer, centrifugation was performed twice, the supernatant was removed, and 200 µL fixation/permeabilization solution was added, followed by incubation at 4°C for 30 minutes. After centrifugation, the supernatant was removed, 200 µL of 1 x Perm wash buffer was added, followed by centrifugation. After 100 µL of 1 x Perm wash buffer was added per well, and then anti-human IFN-η FITC (1 µL) and anti-human TNF-α PE-Cy7 (1 µL) were added to each well, followed by incubation at 4°C for 30 minutes. 100 µL of 1 x Perm wash buffer was added, followed by centrifugation, and the supernatant was removed. Then, 200 µL of 1 x Perm wash buffer was added, followed by centrifugation. The supernatant was removed, 200 µL of BD Cytofix was added, measurements were performed using the LSRFortessa FACS system. The results were analyzed using the FlowJo analysis program. The expression of CD107a and cytokines (IFN-γη and TNF-α) toward the K562 cell line, which was used to evaluate the activity of NK cells, was high in CBNK and CD5 CAR-NK without significant difference. In addition, when the NK cells were co-cultured with the positive cell lines CCRF-CEM and RPMI-8402_Luc cell lines, the expression of CD107a and cytokines (IFN-γη and TNF-α) was at least two-fold higher in the CD5 CAR-NK group than in CBNK, whereas the expression was at a low level in the negative cell line NALM-6, indicating that the cytokines were expressed CD5-specific manner (FIG. 9). The results of measuring the expression levels of CD107a and two cytokines in three donors were assessed for statistical significance using a two-tailed t-test. The results are shown as mean value ± standard deviation in Table 8 below (FIG. 9, *p<0.05, **p<0.01, ns: not significant). The CD-specific expression of CD107a and the cytokines was higher in CD5 CAR-NK in CBNK, and there was no statistical significance between MCB and DP.

**[Table 8] Mean expression of CD107α, IFN-η and TNF-α (n=3)**

| CD107α | | w/o target | K562 | NALM6 | CCRF-CEM | RPMI-8402_ Luc |
|---|---|---|---|---|---|---|
| CBNK (MCB) | Mean | 5.59 | 55.57 | 10.26 | 18.19 | 7.87 |
| | STD | 8.48 | 14.85 | 11.58 | 8.79 | 7.99 |
| CD5 CAR-NK (MCB) | Mean | 7.97 | 48.55 | 9.15 | 45.37 | 30.6 |
| | STD | 7.68 | 3.14 | 7.05 | 7.17 | 13.82 |
| p-value of CBNK (MCB) vs. CD5CAR-NK (MCB) | | ns | ns | ns | 0.0149 | 0.0643 |
| CBNK (DP) | Mean | 4.14 | 46.69 | 7.96 | 19.82 | 5.72 |
| | STD | 5.44 | 5.19 | 3.23 | 3.02 | 4.07 |
| CD5 CAR-NK (DP) | Mean | 7.56 | 47.51 | 9.87 | 43.15 | 24.4 |
| | STD | 9.53 | 6.79 | 4.41 | 7.19 | 8.56 |
| p-value of CBNK (DP) vs. CD5CAR-NK(DP) | | ns | ns | ns | 0.0429 | 0.0251 |
| p-value of CDSCAR-NK (MCB) vs. CD5CAR-NK(DP) | | ns | ns | ns | 0.0573 | 0.4914 |
| IFN-γ | | w/o target | K562 | NALM6 | CCRF-CEM | RPMI-8402_ Luc |
| CBNK (MCB) | Mean | 3.3 | 56.51 | 4.66 | 9.89 | 3.76 |
| | STD | 2.83 | 6.62 | 2.37 | 0.2 | 0.98 |
| CD5 CAR-NK | Mean | 7.65 | 50.99 | 7.8 | 54.7 | 45.47 |
| (MCB) | STD | 6.36 | 15.79 | 5.47 | 14.86 | 15.82 |
| p-value of CBNK (MCB) vs. CD5CAR-NK (MCB) | | ns | ns | ns | 0.0344 | 0.05 |
| CBNK (DP) | Mean | 5.17 | 49.83 | 5.96 | 13.65 | 4.23 |
| | STD | 4.91 | 7.1 | 2.6 | 5.25 | 1.67 |
| CD5 CAR-NK (DP) | Mean | 3.17 | 49.54 | 5.36 | 49.88 | 36.07 |
| | STD | 1.07 | 8.15 | 1.71 | 5.35 | 3.91 |
| p-value of CBNK (DP) vs. CD5CAR-NK (DP) | | ns | ns | ns | 0.0064 | 0.0072 |
| p-value of CDSCAR-NK (MCB) vs. CD5CAR-NK (DP) | | ns | ns | ns | 0.4959 | 0.3298 |
| TNF-α | | w/o target | K562 | NALM6 | CCRF-CEM | RPMI-8402_ Luc |
| CBNK (MCB) | Mean | 3.49 | 44.49 | 7.54 | 10.27 | 5.45 |
| | STD | 4.78 | 18.61 | 8.03 | 6.53 | 5.91 |
| CD5 CAR-NK (MCB) | Mean | 4.38 | 35.72 | 5.94 | 41.33 | 37.24 |
| | STD | 3.9 | 3.09 | 4.5 | 3.76 | 5.18 |
| p-value of CBNK (MCB) vs. CD5CAR-NK (MCB) | | ns | ns | ns | 0.0042 | 0.0024 |
| CBNK (DP) | Mean | 3.82 | 41.66 | 6.83 | 12.94 | 5.14 |
| | STD | 5.15 | 4 | 3.54 | 2.22 | 4.09 |
| CD5 CAR-NK (DP) | Mean | 3.83 | 42.28 | 7.59 | 45.47 | 35.34 |
| | STD | 4.47 | 5.3 | 3.61 | 0.46 | 2.81 |
| p-value of CBNK (DP) vs. CD5CAR-NK (DP) | | ns | ns | ns | 0.0022 | 0.0018 |
| p-value of CDSCAR-NK (MCB) vs. CD5CAR-NK (DP) | | ns | ns | ns | 0.2001 | 0.4893 |

### 6-4) Evaluation of Tumor Cell Killing Ability of CD5 CAR-Transduced Cells

NK cells (CBNK, CD5 CAR-NK) were recovered and centrifuged 1,200 rpm at room temperature for 5 minutes. The supernatant was removed, and the cells were suspended in 1 mL assay medium and counted. The transduced cells were suspended in the assay medium at each E (NK cell): T (target cell) ratio and added to 5-mL FACS tubes. The NK cells were prepared at 1 x 10⁵ cells/100 µL for a 10:1 ratio, 3 x 10⁴ cells/100 µL for a 3:1 ratio, 1 x 10⁴ cells/100 µL for a 1:1 ratio, and 3 x 10³ cells/100 µL for a 0.3:1 ratio.

The target cell line was fixed at 1 x 10⁴ cells/well (1 x 10⁵ cells/mL, 100 µL), and 100 µL of the target cell line and 100 µL of NK cells were added to each well of a 96-well round bottom flask at indicated E:T ratio. To adjust the fluorescence value of Calcein-AM released from the target cells stained with Calcein-AM, the value measured by addition of 100 µL target cells and 100 µL assay medium was taken as the minimum release value. To measure the maximum fluorescence value of Calcein-AM released from the target cells, 100 µL target cells and 100 µL 2% Triton X-100 were added to completely lyse the cells, and the fluorescence value measured at this time was taken as the maximum release value. In order to correct the decrease of the fluorescence value by Triton X-100, 200 µL assay medium was added to each MM well, and 100 µL of assay medium and 100 µL of 2% Triton X-100 were added to each MT well. The plate was incubated in a 37°C incubator for 4 hours under light-shielded conditions, followed by centrifugation at 2,000 rpm and 4°C for 3 minutes. 100 µL of the supernatant was transferred into a black 96-well flat bottom plate, and the fluorescence value was measured using a fluorometer under the conditions of 458 nm/535 nm and 0.1s. The cell killing ability was calculated according to the formula shown in Table 9 below.

**[Table 9] Calculation of killing ability of NK cells**

| Method for calculating killing ability of NK cells |
|---|
| Mean fluorescence value of MM well - mean fluorescence value of MT wells = specific lysis rate A% = [(mean fluorescence value of sample wells - mean fluorescence value of wells with |
| minimum release) /(mean fluorescence value of wells with maximum release + A) - mean fluorescence value of wells with minimum release] x 100 |

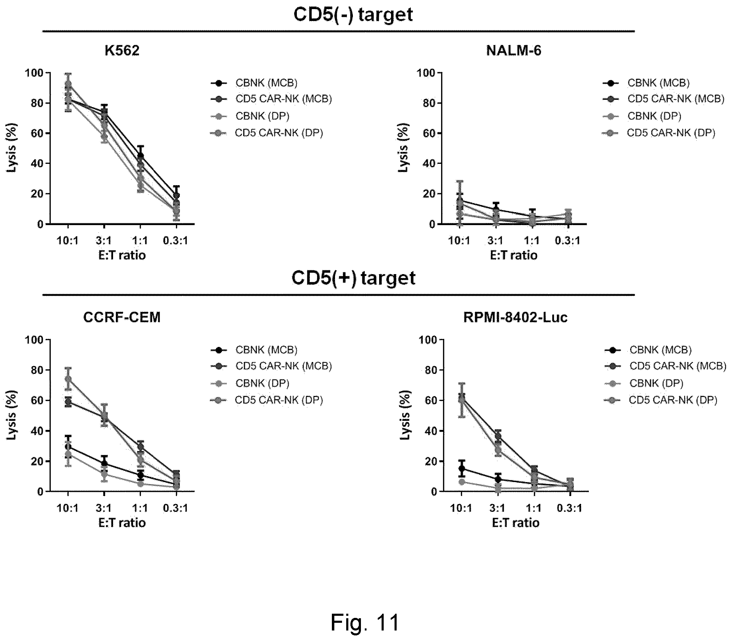

The cell killing ability of each of CBNK (MCB), CD5 CAR-NK (MCB), CBNK (DP), and CD5 CAR-NK (DP) against four types of target cells from 3 donors (2024P, 2044P, and 605463P) is shown in FIG. 11, and each cell killing activity value (%) is summarized in Table 10 below. As shown in FIG. 11 and Table 10, the tumor killing activity of CD5 CAR-NK cells against CCRF-CEM and RPMI-8402-Luciferase cells, which are CD5-positive cell lines, was higher than that of CBNK, and the difference in tumor killing ability between CD5 CAR-NK (MCB) and CD5 CAR-NK (DP) was not significant (2-tailed t-test, *p<0.05, **p<0.01, ***<0.001, ns: not significant).

**[Table 10] Killing ability (%) against cells from three donors (2024P, 2044P, and 605463P)**

| K562 | | 10:1 | 3:1 | 1:1 | 0.3:1 |
|---|---|---|---|---|---|
| CBNK (MCB) | Mean | 82.65 | 74.02 | 45.16 | 18.80 |
| | STD | 2.96 | 4.81 | 6.25 | 6.08 |
| CD5 CAR-NK (MCB) | Mean | 82.53 | 71.45 | 39.17 | 14.13 |
| | STD | 7.87 | 4.24 | 4.08 | 5.14 |
| p-value of CBNK (MCB) vs. CD5 CAR-NK (MCB) | | ns | ns | ns | ns |
| CBNK (DP) | Mean | 82.13 | 57.72 | 25.43 | 8.28 |
| | STD | 6.74 | 3.83 | 4.27 | 2.65 |
| CD5 CAR-NK (DP) | Mean | 92.76 | 65.25 | 30.27 | 8.26 |
| | STD | 6.61 | 7.40 | 8.32 | 5.59 |
| p-value of CBNK (DP) vs. CD5 CAR-NK (DP) | | ns | ns | ns | ns |
| p-value of CD5 CAR-NK (MCB) vs. CD5 CAR-NK (DP) | | ns | ns | ns | 0.0264 |
| NALM6 | | 10:1 | 3:1 | 1:1 | 0.3:1 |
| CBNK (MCB) | Mean | 15.60 | 9.48 | 5.31 | 3.54 |
| | STD | 4.27 | 4.44 | 4.25 | 2.93 |
| CD5 CAR-NK (MCB) | Mean | 6.75 | 2.91 | 0.43 | -0.75 |
| | STD | 3.14 | 3.04 | 3.89 | 2.95 |
| p-value of CBNK (MCB) vs. CD5CAR-NK (MCB) | | 0.0286 | 0.0226 | 0.0242 | 0.0291 |
| CBNK (DP) | Mean | 6.96 | 3.11 | 3.80 | 6.78 |
| | STD | 7.68 | 3.66 | 2.79 | 2.59 |
| CD5 CAR-NK (DP) | Mean | 13.51 | 3.45 | 1.75 | 3.95 |
| | STD | 14.69 | 5.43 | 2.19 | 2.60 |
| p-value of CBNK (DP) vs. CDSCAR-NK (DP) | | ns | ns | 0.045 | 0.0017 |
| p-value of CD5 CAR-NK (MCB) vs. CD5 CAR-NK (DP) | | ns | ns | ns | ns |
| CCRF-CEM | | 10:1 | 3:1 | 1:1 | 0.3:1 |
| CBNK (MCB) | Mean | 29.68 | 18.56 | 10.86 | 4.83 |
| | STD | 7.08 | 4.96 | 3.05 | 1.64 |
| CD5 CAR-NK (MCB) | Mean | 59.04 | 48.76 | 29.66 | 11.09 |
| | STD | 2.83 | 2.59 | 3.54 | 2.43 |
| p-value of CBNK (MCB) vs. CD5CAR-NK (MCB) | | 0.0345 | 0.0182 | 0.0378 | ns |
| CBNK (DP) | Mean | 24.91 | 11.53 | 5.16 | 2.98 |
| | STD | 7.86 | 4.65 | 0.74 | 0.74 |
| CD5 CAR-NK | Mean | 74.20 | 50.30 | 20.90 | 6.86 |
| (DP) | STD | 7.06 | 7.02 | 4.33 | 3.68 |
| p-value of CBNK (DP) vs. CDSCAR-NK (DP) | | 0.0002 | 0.0063 | 0.0237 | ns |
| p-value of CD5 CAR-NK (MCB) vs. CD5 CAR-NK (DP) | | ns | ns | 0.0437 | ns |
| RPMI-8402 luciferase | | 10:1 | 3:1 | 1:1 | 0.3:1 |
| CBNK (MCB) | Mean | 15.27 | 8.13 | 5.17 | 3.52 |
| | STD | 5.29 | 3.71 | 2.66 | 4.91 |
| CD5 CAR-NK (MCB) | Mean | 61.76 | 36.60 | 13.97 | 3.29 |
| | STD | 2.37 | 3.78 | 2.73 | 4.45 |
| p-value of CBNK (MCB) vs. CD5 CAR-NK (MCB) | | 0.0082 | 0.0044 | 0.0051 | ns |
| CBNK (DP) | Mean | 6.44 | 2.26 | 2.09 | 4.90 |
| | STD | 0.77 | 2.60 | 3.48 | 3.61 |
| CD5 CAR-NK (DP) | Mean | 60.12 | 27.42 | 9.28 | 4.86 |
| | STD | 11.07 | 3.84 | 2.82 | 3.38 |
| p-value of CBNK (DP) vs. CD5 CAR-NK (DP) | | 0.0129 | 0.0144 | 0.0028 | ns |
| p-value of CD5 CAR-NK (MCB) vs. CD5 CAR-NK (DP) | | ns | 0.0025 | ns | ns |

### 6-5) Evaluation of IFN-γ Secretion Ability of CD5 CAR-Transduced Cells

Target cells were prepared at a concentration of 1 x 10⁵ cells/mL by dilution in assay medium, and NK cells were prepared at a concentration of 3 x 10⁵ cells/mL by dilution in assay medium. 100 µL of the target cells and 100 µL of the NK cells were dispensed into a 96-well round bottom flask and co-cultured at a ratio of E:T = 3:1. After the cells were cultured in a 5% CO₂ incubator at 37°C for 24 hours, the supernatant was collected and stored at -20°C.

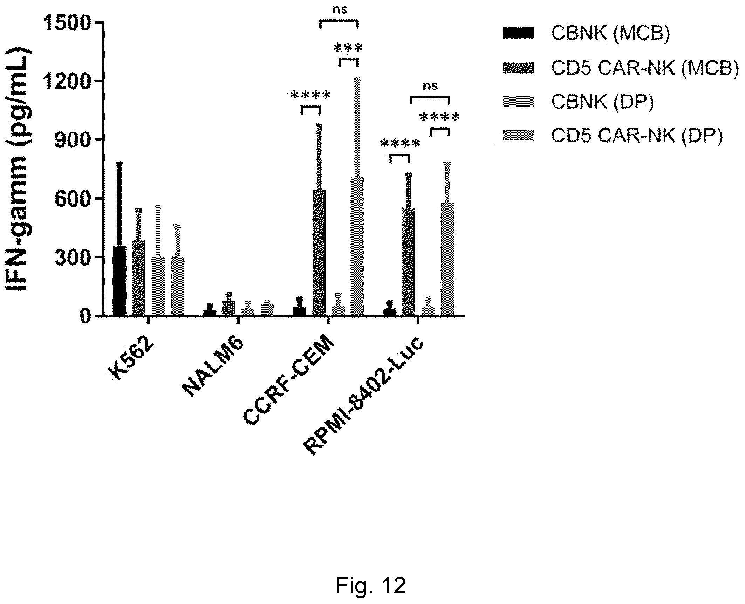

ELISA was performed to quantify IFN-γ. One day before the experiment, 200x capture antibody was diluted in 1x coating buffer A, and then 100 µL of the dilution was added to a 96-well plate, followed by incubation at 2 to 8°C for 16 to 18 hours. On the day of the experiment, the plate was washed four times with wash buffer, and 200 µL of 1x Assay Diluent A was added to each well to suppress non-specific binding. The plate was sealed and left on a plate shaker for 1 hour. A standard was prepared by stepwise dilution of interferon-gamma standard stock solution in 1x Assay Diluent A (500, 250, 125, 62.5, 31.3, 15.6, 7.8, and 0 pg/mL). Thereafter, the supernatant stored at -20°C was thawed and diluted 1/10 in 1 x Assay Diluent A, thus preparing a sample. 100 µL of the standard and 100 µL of the diluted sample were dispensed into each well, and the plate was sealed and incubated on a plate shaker at room temperature for 2 hours. The plate was washed four times with wash buffer. 100 µL of 1x detection antibody was added to each well, and the plate was sealed and incubated on a plate shaker at room temperature for 1 hour. The plate was washed four times with wash buffer. 100 µL of 1x Avidin-HRP was added to each well, and the plate was sealed and incubated on a plate shaker at room temperature for 30 minutes. After washing the plate five times with wash buffer, 100 µL of TMB substrate was added to each well, followed by incubation at room temperature for 20 minutes under light-shielded conditions. The reaction was terminated by adding 100 µL of stop solution to each well, and the absorbance at a wavelength of 450 nm was measured using a spectrophotometer. As a result, in the CD5-negative cell lines K562 and NALM6, there was a difference in the secretion of IFN-γ between CBNK (MCB, DP) and CD5 CAR-NK (MCB, DP) from three donors (2024P, 2044P, and 605463P), however, there was no significant difference when the results for all the donors were analyzed collectively. In the CD5-positive cell lines CCRF-CEM and RPMI-8402-Luciferase, CBNK (MCB, DP) from donor 2024P secreted 40 to 60 pg/mL of IFN-γ, and CD5 CAR-NK (MCB, DP) secreted 350 to 550 pg/mL of IFN-γ, suggesting that the amount of IFN-γ secreted by CD5 CAR-NK was about 8 to 9 times higher than that by CBNK. In the case of donor 2044P, CBNK (MCB, DP) secreted 45 to 55 pg/mL of IFN-γ, and CD5 CAR-NK (MCB, DP) secreted 250 to 350 pg/mL, indicating that CD5 CAR-NK secreted about 5 to 6 times more IFN-γ than CBNK. In the case of donor 605463P, CBNK (MCB, DP) secreted 70 to 120 pg/mL of IFN-γ, and CD5 CAR-NK (MCB, DP) secreted 600 to 1,300 pg/mL of IFN-γ, indicating that CD5 CAR-NK secreted 8 to 10 times more IFN-γ than CBNK. Taken together, CD5 CAR-NK from all of the three donors exhibited improved IFN-γ secretion ability in a manner specific to the CD5-positive cell lines, and showed statistically significant results (FIG. 12, *p<0.05, **p<0.01, ***<0.001, ****<0.0001, ns: not significant). There was no statistical significance between CD5 CAR-NK (MCB) and CD5 CAR-NK (DP).

**[Table 11] IFN-γ secretion (pg/mL) of NK cells from three donors**

| IFN-g | | K562 | NALM6 | CCRF-CEM | RPMI-8402_Luc |
|---|---|---|---|---|---|
| CBNK (MCB) | Mean | 357.54 | 31.32 | 45.56 | 37.56 |
| | STD | 365.59 | 21.42 | 37.40 | 27.52 |
| CD5 CAR-NK (MCB) | Mean | 386.74 | 76.47 | 646.38 | 556.69 |
| | STD | 146.29 | 30.05 | 285.06 | 156.63 |
| p-value of CBNK (MCB) vs. CD5 CAR-NK (MCB) | | ns | 0.0021 | <0.0001 | <0.0001 |
| CBNK (DP) | Mean | 304.12 | 37.84 | 54.67 | 45.59 |
| | STD | 228.44 | 25.69 | 46.72 | 37.87 |
| CD5 CAR-NK (DP) | Mean | 303.88 | 59.91 | 707.42 | 581.36 |
| | STD | 142.75 | 9.07 | 443.92 | 194.58 |
| p-value of CBNK (DP) vs. CD5 CAR-NK (DP) | | ns | 0.0272 | 0.0005 | <0.0001 |
| p-value of CD5 CAR-NK (MCB) vs. CD5 CAR-NK (DP) | | ns | ns | ns | ns |

### 6-6) Evaluation of IL-15 Secretion Ability of CD5 CAR-Transduced Cells

Target cells and NK cells were prepared at concentrations of 2 x 10⁶ cells/mL and 6 x 10⁶ cells/mL, respectively, by dilution in assay media. 100 µL of the target cells and 100 µL of the NK cells were dispensed into a 96-well round bottom flask at a ratio of E:T = 1:3 and co-cultured in a 5% CO₂ incubator at 37°C for 24 hours. Then, the supernatant was collected and stored at -20°C.

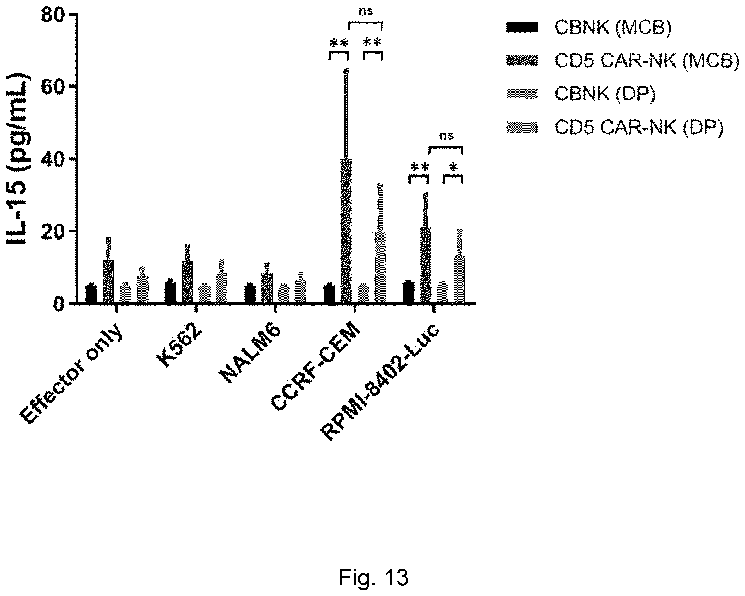

ELISA assay was performed using the IL-15 ELISA kit. The microplate strip was inserted into the plate frame, and 100 µL of the assay diluent RD1-19 was dispensed into each well. 50 µL of the IL-15 standard and 50 µL of the stored supernatant were dispensed onto each well of the plate which was then incubated on a shaker at room temperature for 3 hours. The plate was washed four times with wash buffer. 200 µL of "IL-15 conjugate" was added to each well of the plate which was then incubated on a shaker at room temperature for 45 minutes. After washing the plate four times with wash buffer, 200 µL of "substrate solution" was added to each well of the plate which was then incubated at room temperature for 30 minutes under light-shielded conditions. The reaction was terminated by adding 50 µL stop solution, and the absorbance at a wavelength of 450 nm was measured. As shown in FIG. 13 and Table 12 below, under conditions where the NK cells were cultured alone without the target cells (effector only), CBNK (MCB, DP) from the three donors secreted 4 to 6 pg/mL of IL-15, and CD5 CAR-NK (MCB, DP) secreted 5 to 17 pg/mL of IL-15. In the CD5-negative cell lines K562 and NALM6, CBNK (MCB, DP) and CD5 CAR-NK (MCB, DP) from the three donors secreted IL-15 at levels similar that in the group in which the NK cells were cultured only (effector only). In the CD5-positive cell lines CCRF-CEM and RPMI-8402-Luciferase, CBNK (MCB, DP) secreted IL-15 at levels similar to those in the CD5-negative cell lines, and the secretion of IL-15 by CD5 CAR-NK (MCB, DP) increased to 25 to 60 pg/mL for donor 2024P, 8 to 13 pg/mL for donor 2044P, and 20 to 50 pg/mL for donor 605463P, indicating that both MCB and DP showed statistically significant results in CD5 CAR-NK compared to CBNK. There was no statistical significance between CD5 CAR-NK (MCB) and CD5 CAR-NK (DP) (FIG. 13, *p<0.05, **p<0.01, ns: not significant).

**[Table 12] IL-15 secretion by MCB and DP (pg/mL)**

| IL-15 | | Effector only | K562 | NALM6 | CCRF-CEM | RPMI-8402_Luc |
|---|---|---|---|---|---|---|
| CBNK (MCB) | Mean | 4.97 | 5.88 | 4.98 | 5.06 | 5.81 |
| | STD | 0.24 | 0.59 | 0.24 | 0.28 | 0.29 |
| CD5 CAR-NK (MCB) | Mean | 12.21 | 11.80 | 8.41 | 39.97 | 20.95 |
| | STD | 5.00 | 3.78 | 2.23 | 21.81 | 8.32 |
| p-value of CBNK (MCB) vs. CD5 CAR-NK (MCB) | | 0.0053 | 0.0036 | 0.0038 | 0.0029 | 0.0012 |
| CBNK (DP) | Mean | 4.97 | 4.99 | 4.95 | 4.86 | 5.58 |
| | STD | 0.41 | 0.16 | 0.12 | 0.25 | 0.20 |
| CD5 CAR-NK (DP) | Mean | 7.65 | 8.55 | 6.59 | 19.85 | 13.27 |
| | STD | 1.86 | 2.89 | 1.58 | 11.39 | 5.98 |
| p-value of CBNK (DP) vs. CD5CAR-NK (DP) | | 0.0063 | 0.0131 | 0.0302 | 0.0092 | 0.0103 |
| p-value of CDSCAR-NK (MCB) vs. CDSCAR-NK (DP) | | ns | ns | ns | ns | ns |

### 6-7) Evaluation for Long-Term Tumor Cell Killing Activity of NK Cells Transduced with CD5 CAR Gene

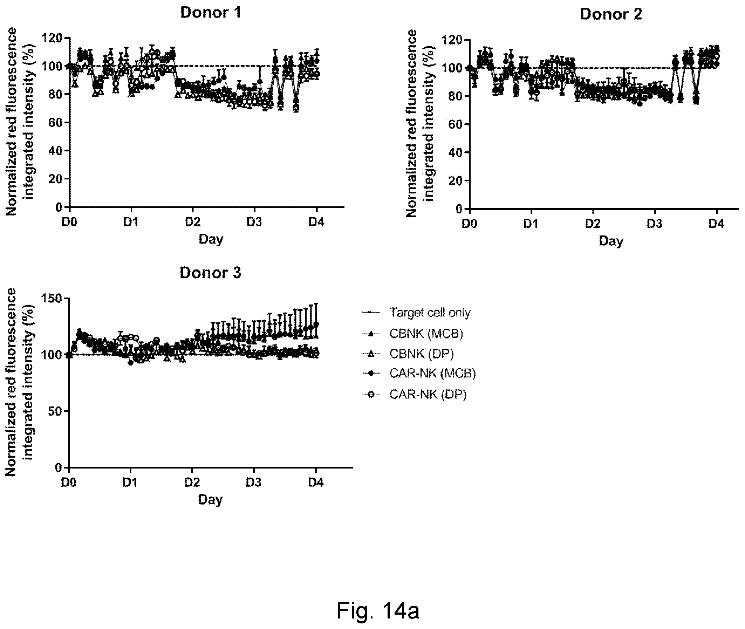

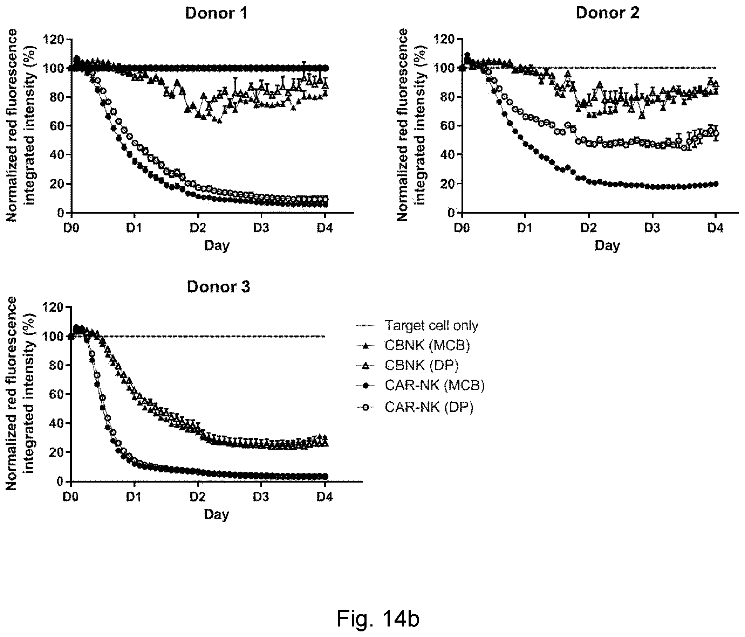

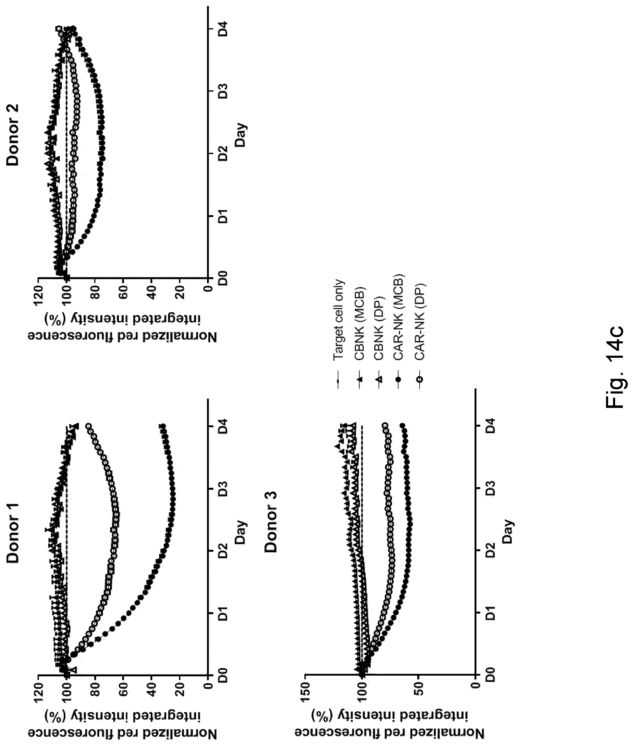

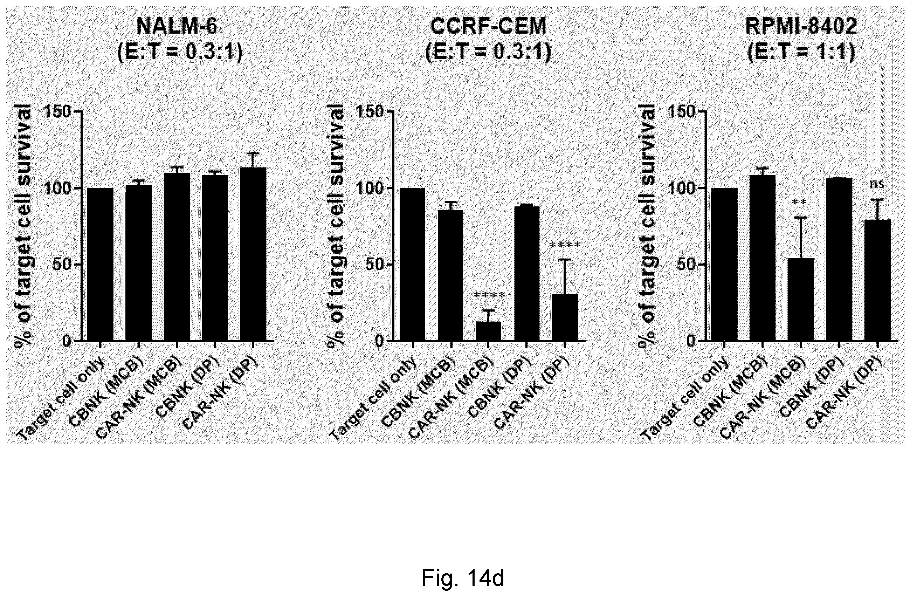

To measure the long-term tumor cell line killing ability of NK cells transduced with the CD5 CAR gene, tumor cell lines expressing red fluorescent protein were co-cultured with CD5 CAR cells from three donors for 96 hours. NALM6, a CD5-negative B cell-derived cancer cell line, and CCRF-CEM and RPMI-8402, CD5-positive acute T lymphoblastic leukemia cell lines, were used in the experiment. Tumor cells were suspended at a concentration of 1 x 10⁵ cells/mL in assay medium (RPMI 1640 (Gibco, 11875093) containing 10% FBS). To make the tumor cell line in a spheroidal form, 100 µL of the tumor cells were added to each well of a 96-well ultra-low attachment (ULA) plate (Corning) and then centrifuged at 125g and 4°C for 10 minutes. NK cells to be co-cultured with NALM6 or RPMI-8402 were prepared at a concentration of 1 x 10⁵ cells/mL (E:T ratio = 1:1) by dilution in assay medium. NK cells to be co-cultured with CCRF-CEM were prepared at a concentration of 0.3 x 10⁵ cells/mL (E:T ratio= 0.3:1) by dilution in assay medium. Then, 100 µL NK cells were added to each well to which the tumor cells had been added. In the control group where the tumor cells were cultured only, 100 µL assay medium instead of the NK cells, was added to each well together with 100 µL of the tumor cells. The plate was placed in the Incucyte S3 instrument (Sartorius), and the red fluorescence intensity (All Brightfield Object Total Red Integrated Intensity, RCU x µm²/Image) in the tumor cell spheroids obtained by 96 hours of co-culture was analyzed using the Oncocyte spheroid analysis program (v2019B). The graphs in FIG. 14 show the values obtained by normalizing the red fluorescence intensity of the co-cultured wells at each time point to the red fluorescence intensity of the wells containing only the tumor cells. As shown in FIG. 14, the tumor killing ability of the NK cells isolated and produced from three donors against the CD5-negative NALM6 cells was similar to that of CBNK cells. For CCRF-CEM, a CD5-positive tumor cell line, CD5 CAR-NK cells (MCB, DP) more consistently inhibited the growth of the tumor cells than CBNK cells (MCB, DP). CD5 CAR-NK cells (MCB) and CD5 CAR-NK cells (DP) from donor 1 had a similar ability to inhibit tumor cell proliferation. On the other hand, in case of the cells from donors 2 and 3, the tumor cell killing ability of CD5 CAR-NK cells (MCB) was higher than that of CD5 CAR-NK cells (DP) when co-cultured with CCRF-CEM. For RPMI-8402, a CD5 positive tumor cell line, in case of the cells from two donors (donors 1 and 3), the tumor cell killing ability of CD5 CAR-NK cells (MCB) versus CBNK cells (MCB, DP) was higher than that of CD5 CAR-NK cells (DP), but CD5 CAR-NK cells (DP) from donor 2 did not show tumoricidal activity (FIG. 14). Significance was assessed through a two-tailed t-test (*p<0.05, **p<0.01, ***<0.001, ns: not significant). In conclusion, the long-term tumor cell killing ability of CD5 CAR NK cells against CD5-positive tumor cells was confirmed, and CD5 CAR-NK (MCB) cells showed higher killing ability than CD5 CAR-NK (DP) cells (Table 13).

**[Table 13] Long-term tumor cell-killing ability of CD5 CAR NK cells**

| | | NALM-6 (0.3:1) | CCRF-CEM (0.3:1) | RPMI-8402 (1:1) |
|---|---|---|---|---|
| Target only | Mean | 100 | 100 | 100 |
| | STD | 0 | 0 | 0 |
| CBNK (MCB) | Mean | 102.00 | 85.88 | 108.41 |
| | STD | 7.71 | 5.47 | 5.44 |
| p-value of CBNK (MCB) | | ns | ns | ns |
| CAR-NK (MCB) | Mean | 109.92 | 12.82 | 54.08 |
| | STD | 12.05 | 6.51 | 23.27 |
| p-value of CAR-NK (MCB) | | ns | <0.0001 | 0.0063 |
| CBNK (DP) | Mean | 108.50 | 88.07 | 106.36 |
| | STD | 5.33 | 5.52 | 2.23 |
| p-value of CBNK (DP) | | ns | ns | ns |
| CAR-NK (DP) | Mean | 114.07 | 30.45 | 79.49 |
| | STD | 14.77 | 22.56 | 11.52 |
| p-value of CAR-NK (DP) | | 0.0157 | <0.0001 | ns |

### Example 7: In Vivo Evaluation for Efficacy of CD5 CAR-Transduced Cells

### 7-1) Culture and Freezing of Tumor Cells

The RPMI-8402-Luc cell line was generated by inserting the CML-Luc lentiviral vector into RPMI-8402, a human T-cell acute lymphoblastic leukemia cancer cell line. As a culture medium for the cancer cell line, IMDM medium (ATCC) containing 10% (v/v) fetal bovine serum (FBS) (Gibco) and 2.0 µg/mL of puromycin (Gibco) was used. The cancer cells were adjusted to a concentration of 1 x 10⁵ cells/mL and cultured in a culture vessel with an appropriate volume. A cancer cell line culture medium containing 20% DMSO (Avantor) was used for freezing, and the cancer cell line was stored within a week in an ultra-low temperature freezer using Mr. Frosty (freezing vessel) (Thermo Fisher) and then transferred to and stored in an ultra-low-temperature liquid nitrogen tank.

### 7-2) Xenograft of Tumor Cell and Administration of NK Cells

6-week-old female specific pathogen-free (SPF) NOD.Cg-Prkdc^{scid}IL2γg^{tm1Sug}/JicKoat mice (hereinafter referred to as NOG) (supplied by Saeron Bio) (produced by Coretech Co., Ltd.)) were used in the experiments. Cancer cells to be used for tumor transplantation were prepared by thawing the frozen cell line and subculturing the cell line once every 3 to 4 days. On the day of transplanting the tumor into mice, all cancer cells were collected, centrifuged, and resuspended in PBS. The experiments for administering tumor cells and NK cells was divided into two parts: ① comparison depending on the number of administrations and dose (MCB), and ② comparison depending on culture conditions (MCB, DP).
① For comparison depending on the number of administrations and dose, a cell suspension of the tumor cell line was prepared at a concentration of 5 x 10⁶ cells/mL. 0.2 mL of the prepared cell suspension per mouse in each experimental group was injected into the tail vein so that the cancer cells were transplanted at 1 x 10⁶ cells/head. The mice were divided into a total of three test groups: (A) a group administered CBNK (MCB) and CD5 CAR-NK (MCB) cells once 3 days after transplantation; (B) a group administered CBNK (MCB) and CD5 CAR-NK (MCB) cells three times at intervals of twice a week starting from 3 days after transplantation; and (C) a group administered CBNK (MCB) and CD5 CAR-NK (MCB) cells three times at intervals of once a week from 3 days after transplantation (Table 14). After thawing the frozen NK cells, the cells were resuspended in freezing medium at the concentrations according to indicated conditions, and then 0.2 mL of each cell suspension was injected into the mouse tail vein once or a total of three times. For a total of three administrations, the NK cells were administered twice a week on days 3, 7, and 10, and once a week on days 3, 10, and 17. For the experimental group administered a total of 3 times a week, the dose of CD5 CAR-NK (MCB) was divided into 2 x 10⁶, 5 x 10⁶, and 1 x 10⁷ cells/head. As control groups, a group administered only PBS and a group administered only the cancer cell line at 1 x 10⁶ cells/head (Tumor-only group) were added. Each group included 3 or 5 animals.
② For comparison depending on culture period (MCB and DP), a cell suspension of the tumor cell line was prepared at a concentration of 1.5 x 10⁷ cells/mL. 0.2 mL of the prepared cell suspension per mouse in each experimental group was injected into the tail vein so that the cancer cells were transplanted at 3 x 10⁶ cells/head. Starting 3 days after transplantation, CBNK (MCB) and CD5 CAR-NK (MCB, DP) cells were administered once at a dose of 1 x 10⁷ cells/head (Table 15). As control groups, a group administered only PBS and a Tumor-only group administered only the cancer cell line at 3 × 10⁶ cells/head were added. Each group included 3 or 5 animals.

**[Table 14] Conditions for NK cell administration (① Comparison depending on number of administrations and dose)**

| Group | Substance administered | Dose (cells/head) | Dosing interval | Number of administrations | Number of animals |
|---|---|---|---|---|---|
| Control group | PBS | N/A | N/A | N/A | 3 |
| | Tumor only | N/A | N/A | N/A | 5 |
| (A) | CBNK (MCB) | 1 x 10⁷ | Once | Once | 5 |
| | CD5 CAR-NK (MCB) | 1 x 10⁷ | Once | Once | 5 |
| (B) | CBNK (MCB) | 1 x 10⁷ | Twice a week | Three times | 5 |
| | CD5 CAR-NK (MCB) | 1 x 10⁷ | Twice a week | Three times | 5 |
| (C) | CBNK (MCB) | 1 x 10⁷ | Once a week | Three times | 5 |
| | CD5 CAR-NK (MCB) | 1 x 10⁷ | Once a week | Three times | 5 |
| | CD5 CAR-NK (MCB) | 5 x 10⁶ | Once a week | Three times | 5 |
| | CD5 CAR-NK (MCB) | 2 x 10⁶ | Once a week | Three times | 5 |

**[Table 15] Conditions for NK cell administration (② Comparison depending on culture period (MCB, DP)**

| Group | Substance administered | Dose (cells/head) | Dosing interval | Number of administrations | Number of animals |
|---|---|---|---|---|---|
| Control group | PBS | N/A | N/A | N/A | 3 |
| | Tumor only | N/A | N/A | N/A | 5 |
| Experim ental group | CBNK (MCB) | 1 x 10⁷ | Once a week | Three times | 4 |
| | CD5 CAR-NK (MCB) | 1 x 10⁷ | Once a week | Three times | 7 |
| | CD5 CAR-NK (DP) | 1 x 10⁷ | Once a week | Three times | 7 |

### 7-3) Evaluation for Efficacy of CD5 CAR-NK Cells in Mice Depending on Number of Administrations and Dose

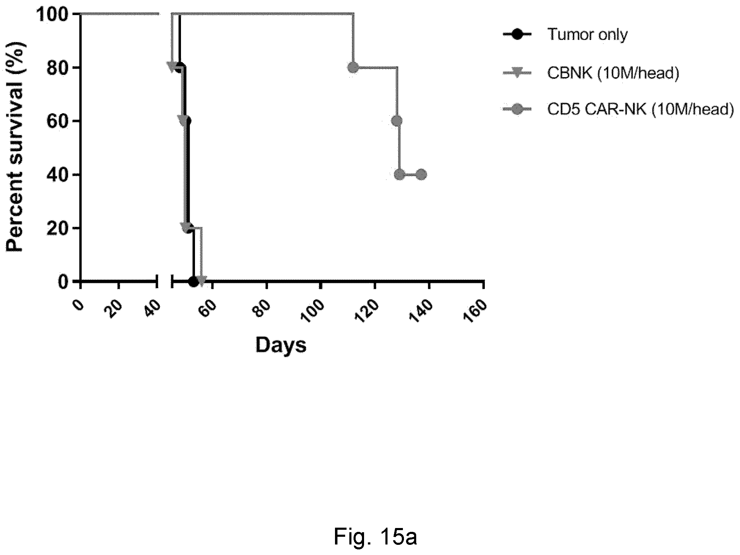

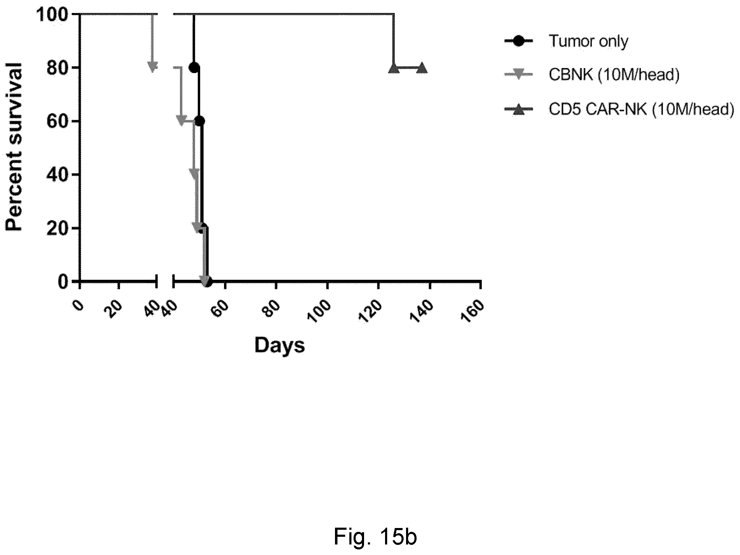

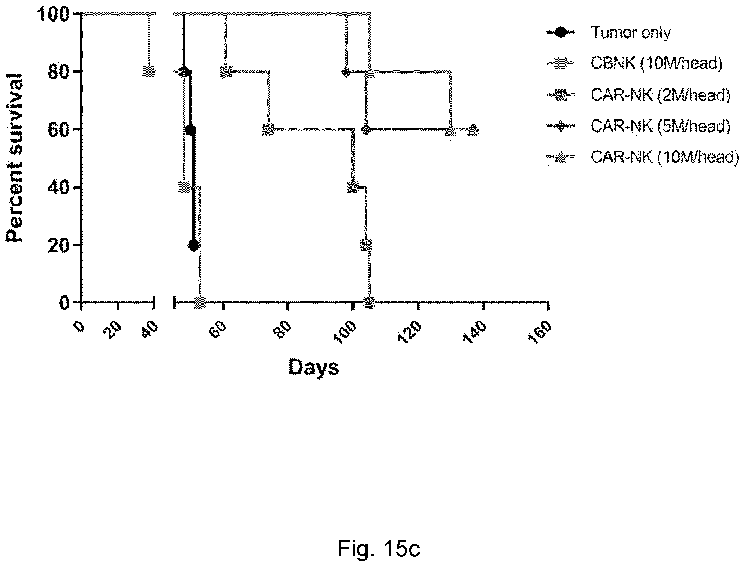

For all animals, general symptoms were measured twice a day (once on weekends and holidays) during the test period, and body weight changes were measured three times a week. Starting from day 60 after cancer cell transplantation, body weight changes were measured once a week. After checking whether the animals died, the survival rate was evaluated by calculating the median survival time for all groups using GraphPad Prism. D-luciferin (GOLDBIO) at a concentration of 15 mg/ml was administered intraperitoneally to each side of the mouse's abdomen (50 µL), and then small-animal bioimaging (Perkin Elmer, IVIS Spectrum Series) was performed under inhalation anesthesia with 2% isoflurane for 7 minutes. Imaging was performed once a week starting from the date of cancer cell transplantation, a total of 13 times (days 0, 7, 14, 21, 28, 35, 42, 49, 56, 70, 84, 102, and 112 from the date of transplantation). As a result, in the CBNK (MCB)-administered group, symptoms of hind limb paralysis were observed in all of the animals before and after day 40 regardless of the number of administrations, and all of the animals died within 2 weeks after symptom onset. The median survival time was measured to be 48 to 50 days because death occurred earlier than in the tumor-only group (Table 16). In the case of the CD5 CAR-NK (MCB)-administered groups, hind limb paralysis symptoms were not observed in the group administered CD5 CAR-NK (MCB) once, and hind limb paralysis symptoms were observed only in some animals in the group administered CD5 CAR-NK (MCB) a total of three times at intervals of twice a week and the group administered CD5 CAR-NK (MCB) a total of three times at intervals of once a week. As a result of comparison depending on dose, more animals showed symptoms of hind limb paralysis when administered at a low dose. At the final observation point (day 137), the median survival times of the CD5 CAR-NK (MCB)-administered groups were 129 days for the group administered once, >137 days for the group administered a total of three times at intervals of twice a week, >137 days for the groups administered a total of three times at intervals of once a week (experimental groups administered at doses of 5 x 10⁶ cells, and 1x10⁷ cells/head), and 100 days for the group administered a total of three times at intervals of once a week (experimental group administered at a dose of 2 x 10⁶ cells/head), which were at least two times longer than the median survival periods of the tumor-only group and the CBNK (MCB)-administered group (50 days) (Table 16). From the results for the groups administered a total of 3 times once a week, it was confirmed that the survival period tended to increase as the dose and number of administrations increased (FIG. 15) (*p<0.05, **p<0.01, ***<0.001, ns: not significant).

In regard to body weight change, the tumor-only group and the CBNK (MCB)-administered group began to lose weight around 37 days. All the animals in these groups died on days 56 and 53, respectively, and showed a tendency to lose weight with the progress of the disease and then die. In the case of the CD5 CAR-NK (MCB)-administered group, some animals died without significant weight loss.

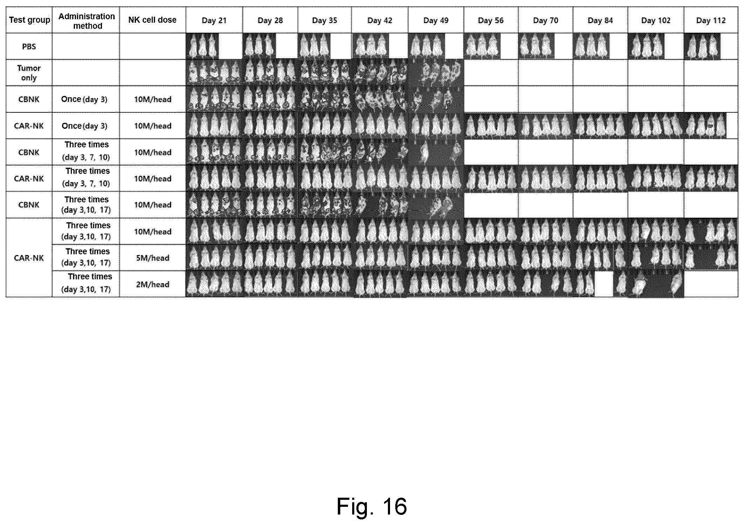

As a result of bio-imaging, bioluminescence was detected starting from day 21, whereas in all the CD5 CAR-NK (MCB)-administered groups, no bioluminescence was not detected, indicating that the CD5 CAR-NK (MCB) exhibited a remarkable inhibitory ability against tumor formation (FIG. 16). It could be confirmed that, even on day 56 when all the animals of the CBNK (MCB)-administered group died, tumor formation was suppressed in the CD5 CAR-NK (MCB)-administered groups, except for some animals in the experimental group administered at 2 x 10⁶ cells/head. As a result of measuring bioluminescence (photons/s), the bioluminescence of the CBNK (MCB)-administered group increased starting from day 21, bioluminescence was maintained at a level similar to that on the day of tumor transplantation until day 35 in all of the groups administered CD5 CAR-NK (MCB) once and three times (FIG. 16). It could be confirmed that, even on day 112, the final imaging time point, the tumor formation inhibitory ability was maintained in the surviving animals of the CD5 CAR-NK (MCB)-administered groups. Statistical significance was assessed through one-way analysis of variance (ANOVA) (*p<0.05, **p<0.01, ***<0.001, ****<0.0001, ns: not significant).

**[Table 16] Median survival time**

| Test group | Substance administered | Dose (cell count per mouse) | Median survival time | P-value |
|---|---|---|---|---|
| Control group | PBS | N/A | >137 days | ns |
| | Tumor only | N/A | 51 days | ns |
| (A) | CBNK (MCB) | 1 x 10⁷ | 50 days | 0.0021 (versus CBNK) |
| | CD5 CAR-NK (MCB) | 1 x 10⁷ | 129 days | |
| (B) | CBNK (MCB) | 1 x 10⁷ | 48 days | 0.0018 (versus CBNK) |
| | CD5 CAR-NK (MCB) | 1 x 10⁷ | >137 days | |
| (C) | CBNK (MCB) | 1 x 10⁷ | 48 days | ns |
| | CD5 CAR-NK (MCB) | 1 x 10⁷ | >137 days | 0.0036 (versus CBNK) |
| | CD5 CAR-NK (MCB) | 5 x 10⁶ | >137 days | 0.0036 (versus CBNK) |
| | CD5 CAR-NK (MCB) | 2 x 10⁶ | 100 days | 0.0026 (versus CBNK) |

**[Table 17] Bioluminescence (photons/s) depending on the number of administrations after tumor transplantation**

| A (photons/s) | | D0 | D7 | D14 | D21 | D28 | D35 |
|---|---|---|---|---|---|---|---|
| CBNK (injected once) | Mean | 242000 | 460000 | 2220000 | 4530000 | 38400000 | 49600000 |
| | SD | 19500 | 132000 | 940000 | 1850000 | 10800000 | 20100000 |
| CAR-NK (injected once) | Mean | 243000 | 459000 | 2020000 | 559000 | 1860000 | 475000 |
| | SD | 22300 | 150000 | 874000 | 81500 | 848000 | 88400 |
| | *p* value | ns | ns | ns | 0.0047 | <0.0001 | 0.0005 |
| CBNK (three times, twice a week) | Mean | 243000 | 436000 | 2210000 | 5270000 | 31800000 | 60800000 |
| | SD | 19000 | 108000 | 826000 | 2070000 | 7340000 | 30700000 |
| | *p* value | ns | ns | ns | ns | ns | ns |
| CAR-NK (three times, twice a week) | Mean | 243000 | 421000 | 2080000 | 489000 | 2330000 | 559000 |
| | SD | 38900 | 114000 | 902000 | 25500 | 1060000 | 56900 |
| | *p* value | ns | ns | ns | 0.004 | <0.0001 | 0.0005 |
| CBNK (three times, once a week) | Mean | 243000 | 433000 | 1860000 | 6230000 | 32400000 | 66300000 |
| | SD | 26900 | 102000 | 503000 | 3050000 | 4060000 | 18900000 |
| | *p* value | ns | ns | ns | ns | ns | ns |
| CAR-NK (three times, once a week) | Mean | 242000 | 340000 | 1850000 | 689000 | 2760000 | 509000 |
| | SD | 23200 | 110000 | 835000 | 75400 | 1420000 | 23900 |
| | *p* value | ns | ns | ns | 0.0063 | <0.0001 | 0.0005 |

**[Table 18] Bioluminescence (photons/s) depending on administration dose after tumor transplantation**

| B (photons/s) | | D0 | D7 | D14 | D21 | D28 | D35 |
|---|---|---|---|---|---|---|---|
| CBNK (10M/head) | Mean | 243000 | 433000 | 1860000 | 6230000 | 32400000 | 66300000 |
| | SD | 26900 | 102000 | 503000 | 3050000 | 4060000 | 18900000 |
| CAR-NK (2M/head) | Mean | 243000 | 349000 | 2040000 | 732000 | 2580000 | 673000 |
| | SD | 29600 | 110000 | 869000 | 115000 | 1330000 | 148000 |
| | *p* value | ns | ns | ns | 0.0001 | <0.0001 | <0.0001 |
| CAR-NK (5M/head) | Mean | 243000 | 422000 | 2670000 | 542000 | 2230000 | 669000 |
| | SD | 25900 | 117000 | 1230000 | 58900 | 893000 | 128000 |
| | *p* value | ns | ns | ns | <0.0001 | <0.0001 | <0.0001 |
| CAR-NK (10M/head) | Mean | 242000 | 340000 | 1850000 | 689000 | 2760000 | 509000 |
| | SD | 23200 | 110000 | 835000 | 75400 | 1420000 | 23900 |
| | *p* value | ns | ns | ns | <0.0001 | <0.0001 | <0.0001 |

### 7-4) Efficacy of CD5 CAR-NK Cells in Mice Depending on Culture Period

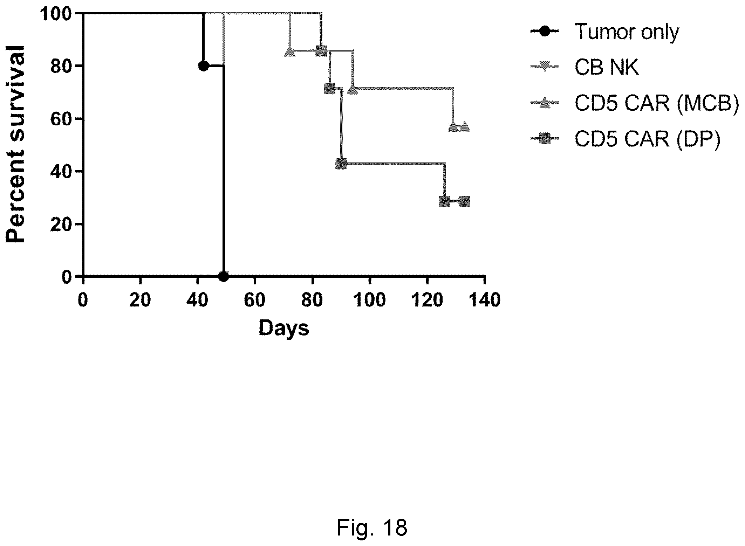

According to the same process as in Example 7-3, general symptoms and body weight changes were measured for all animals twice a week during the experimental period, median survival times were calculated, and survival rates (MCB, DP) were compared depending on the culture period. Imaging was performed once a week starting from the date of cancer cell transplantation, a total of 17 times (days 0, 7, 14, 27, 35, 42, 49, 56, 65, 72, 78, 86, 94, 100, 107, 118, and 126). As results, in the CBNK (MCB)-administered group, symptoms of hind limb paralysis were found around 40 days in all of the animals regardless of the number of administrations All of the animals in this group died within 2 weeks after the onset of the symptoms, and the median survival time of this group was 49 days, which was the same as that of the tumor-only group (Table 19). In the case of the CD5 CAR-NK (MCB)-administered group, hind limb paralysis appeared for the first time on day 69, and one mouse died for the first time on day 72. In the case of the CD5 CAR-NK (DP)-administered group, one mouse died for the first time on day 83, and there was no symptom of hind limb paralysis before death. Unlike the CBNK (MCB)-administered group, symptoms of hind limb paralysis before death were found only in some mice of the CD5 CAR-NK (MCB)-administered group and the CD5 CAR-NK (DP)-administered group. The median survival time was measured to be 90 days for the CD5 CAR-NK (DP)-administered group, but the median survival time of the CD5 CAR-NK (MCB)-administered group could not be calculated because more than half of the mice did not die until day 133 (FIG. 18). Accordingly, the median survival time of the CD5 CAR-NK (MCB)-administered group was longer than that of the CD5 CAR-NK (DP)-administered group, but this value was not statistically significant. However, both the CD5 CAR-NK (MCB)-administered group and the CD5 CAR-NK (DP)-administered group showed a statistically significant improvement in survival rate compared to the CBNK (MCB)-administered group. Statistical significance was assessed through the log-rank (mantel-cox) test (*p<0.05, **p<0.01, ***<0.001, ns: not significant).

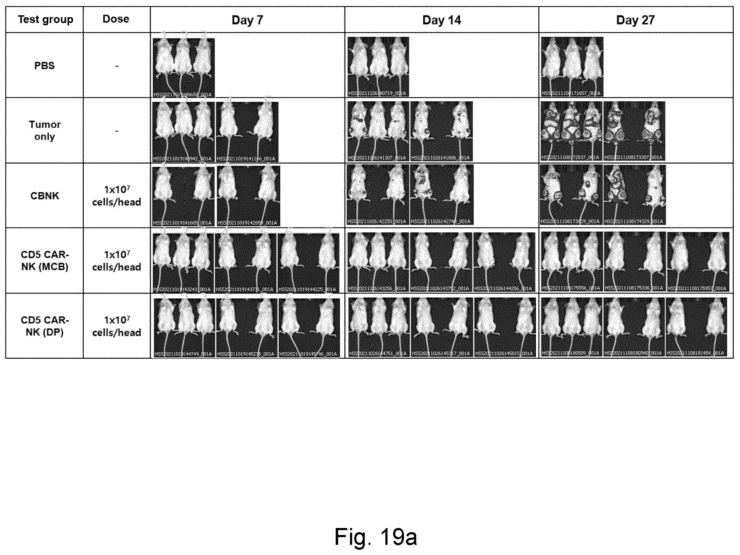

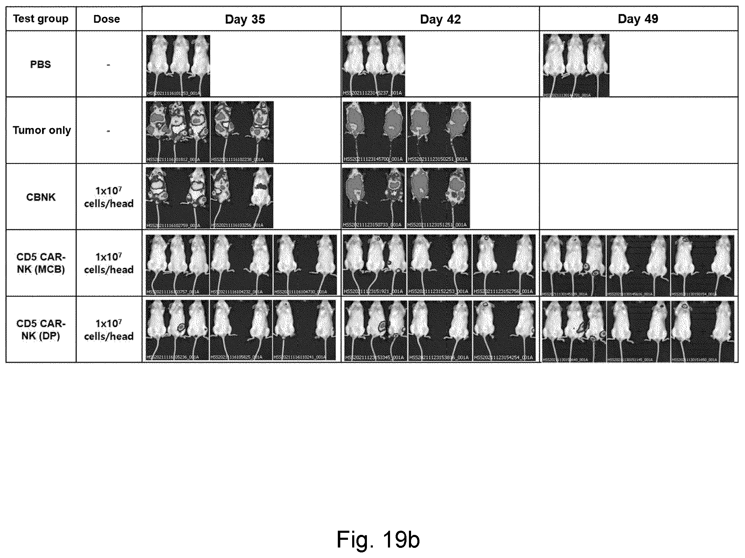

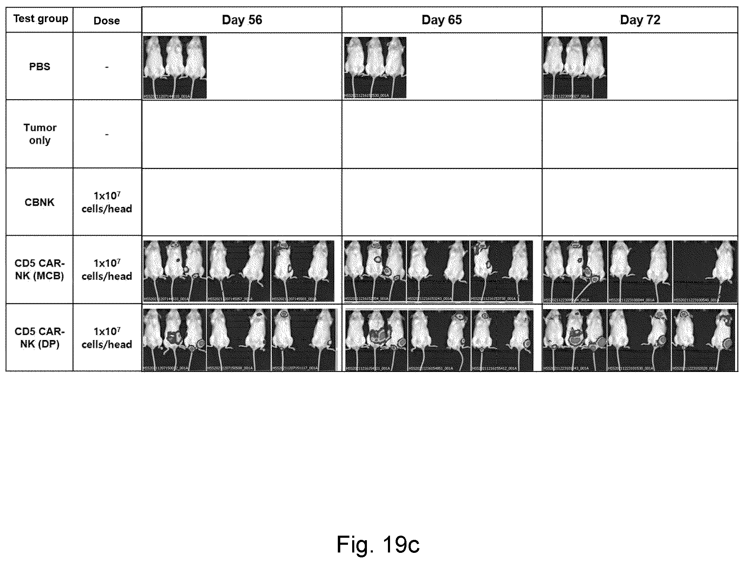

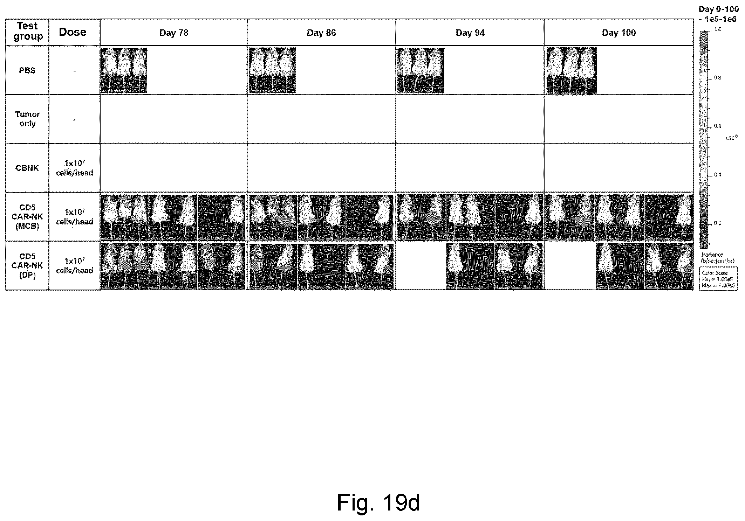

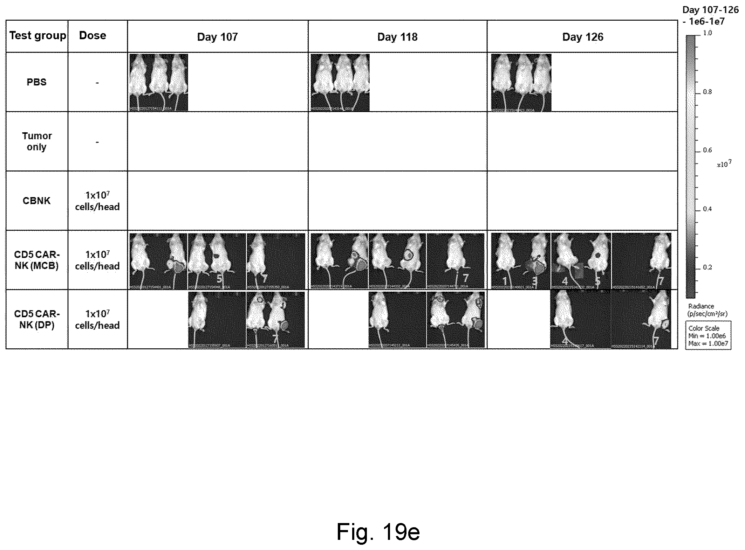

For the tumor-only group and the CBNK (MCB)-administered group, bioluminescence was detected near the hind legs of the mice starting from day 14, whereas in the CD5 CAR-NK (MCB)-administered group, no bioluminescence was detected, indicating that the CD5 CAR-NK (MCB) exhibited a significant inhibitory ability against tumor formation (FIG. 19). In the CD5 CAR-NK (DP)-administered group, bioluminescence began to be detected on day 35 day after tumor cell transplantation. In the CD5 CAR-NK (MCB)-administered group, bioluminescence began to be observed on day 42, a week later. It could be seen that, even on day 49, when all the mice of the CBNK (MCB)-administered group had died, bioluminescence was detected only in some mice in the CD5 CAR-NK (MCB)- and CD5 CAR-NK (DP)-administered groups, indicating that tumor formation was inhibited in the CD5 CAR-NK (MCB, DP)-administered group compared to the CBNK (MCB)-administered group. It was confirmed that the tumor formation inhibitory ability was maintained in the surviving mice even on day 126, the final imaging time point. Statistical significance was assessed through one-way analysis of variance (*p<0.05, **p<0.01, ***<0.001, ****<0.0001, ns: not significant).

**[Table 19] Median survival time**

| Test group | Substance administered | Dose | Median survival time | P-value |
|---|---|---|---|---|
| | | (NK cell count per mice) | | |
| Control group | PBS | N/A | >133 days | ns |
| | Tumor only | N/A | 49 days | ns |
| Experimental group | CBNK (MCB) | 1 x 10⁷ | 49 days | ns |
| | CD5 CAR-NK (MCB) | 1 x 10⁷ | >133 days | 0.0016 (versus CBNK) |
| | CD5 CAR-NK (DP) | 1 x 10⁷ | 90 days | 0.0016 (versus CBNK) |

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A fusion protein comprising:
(a) a chimeric antigen receptor (CAR) comprising an intracellular signaling domain which comprises an OX40 ligand (OX40L), and an extracellular antigen binding domain that binds to CD5; and
(b) interleukin (IL)-15.

2. The fusion protein of claim 1, wherein the extracellular antigen binding domain is an antigen binding fragment of an anti-CD5 antibody.

3. The fusion protein of claim 2, wherein the antigen-binding fragment is an Fab fragment, an F(ab') fragment, an F(ab')2 fragment or an Fv fragment.

4. The fusion protein of claim 2, wherein the extracellular antigen binding domain comprises a heavy chain variable region comprising HCDR1 having the amino acid sequence of SEQ ID NO: 26, HCDR2 having the amino acid sequence of SEQ ID NO: 28, and HCDR3 having the amino acid sequence of SEQ ID NO: 30.

5. The fusion protein of claim 4, wherein the heavy chain variable region comprises the amino acid sequence of SEQ ID NO: 32.

6. The fusion protein of claim 2, wherein the extracellular antigen binding domain further comprises a light chain variable region comprising LCDR1 having the amino acid sequence of SEQ ID NO: 34, LCDR2 having the amino acid sequence of SEQ ID NO: 36, and LCDR3 having the amino acid sequence of SEQ ID NO: 38.

7. The fusion protein of claim 6, wherein the light chain variable region comprises the amino acid sequence of SEQ ID NO: 40.

8. The fusion protein of claim 2, wherein the extracellular antigen binding domain comprises the amino acid sequence of SEQ ID NO: 7

9. The fusion protein of claim 1, wherein the intracellular signaling domain further comprises at least one domain selected from the group consisting of CD28, CD3-zeta, OX40, and 4-1BB.

10. The fusion protein of claim 9, wherein the intracellular signaling domain further comprises CD28 and CD3-zeta.

11. The fusion protein of claim 10, wherein the intracellular signaling domain comprises CD28, OX40L, and CD3-zeta, which are arranged in order from a cell membrane toward an inside of the cell.

12. The fusion protein of claim 1, wherein the fusion protein further comprises a self-cleaving peptide located between the chimeric antigen receptor and the IL-15.

13. The fusion protein of claim 12, wherein the self-cleaving peptide comprises the amino acid sequence of SEQ ID NO: 21.

14. A nucleic acid molecule encoding the fusion protein of any one of claims 1 to 13.

15. An immune cell expressing the nucleic acid molecule of claim 14.

16. An immune cell expressing:
(a) a chimeric antigen receptor (CAR) comprising an intracellular signaling domain which comprises an OX40 ligand (OX40L), and an extracellular antigen binding domain that binds to CD5; and
(b) interleukin (IL)-15.

17. The immune cell of claim 16, wherein the immune cell is a natural killer cell.

18. The immune cell of claim 17, wherein the natural killer cell is a natural killer cell frozen and thawed after culturing for 23 to 35 days.

19. A composition for preventing or treating a CD5-positive tumor, comprising the immune cell of claim 15 or 16 as an active ingredient.

20. The composition of claim 19, wherein the CD5-positive tumor is lymphocytic leukemia.
